# EUROPEAN PATENT APPLICATION

(11) **EP 4 501 391 A1**
(43) Date of publication of application: **05.02.2025**
(21) Application number: 23780593.2
(22) Date of filing: 28.03.2023
(51) Int. Cl.: A61M 37/00

(54) **FINE NEEDLE PART, INJECTION DEVICE, AND PUNCTURE INJECTION SET**

(30) Priority: 30.03.2022 JP 2022056466
(71) Applicant: Shiseido Company, Ltd., Chuo-ku Tokyo 104-0061 (JP); Asti Corporation, Hamamatsu-shi, Shizuoka 432-8056 (JP)
(72) Inventor: SHIMIZU, Hiroko, Tokyo 104-0061 (JP); OBANA, Takakazu, Tokyo 104-0061 (JP); YOSHIMURA, Mika, Tokyo 104-0061 (JP); OGAI, Noriyuki, Hamamatsu-shi, Shizuoka 432-8056 (JP); TODA, Yasuhiro, Hamamatsu-shi, Shizuoka 432-8056 (JP)
(74) Representative: Ter Meer Steinmeister & Partner
(86) International application number: PCT/JP2023/012648
(87) International publication number: WO 2023/190584

(57) **Abstract**

A fine needle part 1 to be attached to a skin puncture instrument includes a plate substrate having a plate shape; a plurality of bases 113 projecting from a surface of the plate substrate, the surface being situated so as to face a target object; and a plurality of needles 114 projecting from top surfaces of the plurality of bases 113. A height (b) of each of the needles of the plurality of needles 114 is 5 µm ≤ b < 700 um. A height (a) of each of the bases of the plurality of bases 113 is 200 µm ≤ a < 3,000 um. A total height (a+b) of the height (b) of each of the needles and the height (a) of each of the bases is from 600 µm through 3,200 µm. The plurality of bases 113 and the plurality of needles 114 are formed of a resin material that does not dissolve. By the target object being punctured by the needles 114, liquid is applied onto a surface of the target object or to an interior of the target object.

## Description

### TECHNICAL FIELD

The present invention relates to a fine needle part, an injection device, and a puncture injection set.

### BACKGROUND ART

In recent years, many cosmetic devices using needles have been developed. For example, a stamp-type needle applicator in which a plurality of needles (microneedles) are arranged in rows, is proposed (see Patent Document 1).

However, when an elastic membrane, such as skin, is punctured by a plurality of needles as described in Patent Document 1, many of the needles cannot readily puncture the skin due to deformation of the skin.

Meanwhile, as a technique of successfully puncturing the skin regardless of deformation of the skin, Patent Document 2 proposes a technique of providing an adjusting portion enclosing the needle and a cylindrical stabilizing portion around the needle.

Also, in recent years, known is a microneedle mask including dissolvable microneedles to be attached to the skin and then left to stand, thereby enabling the beauty ingredients to penetrate into the skin (see, for example, Patent Document 3).

### RELATED ART DOCUMENTS

### PATENT DOCUMENTS

Patent Document 1: Japanese Utility Model Registration No. 3218898
Patent Document 2: Japanese Laid-Open Patent Application No. 2012-029723
Patent Document 3: Japanese Patent No. 6682783

### SUMMARY OF THE INVENTION

### PROBLEMS TO BE SOLVED BY THE INVENTION

The assembled body as in Patent Document 2 deforms the skin by the cylindrical stabilizing portion, so that the skin can be reliably punctured by the needle. However, the needle is provided at only one site at the center, and liquid is applied from this only one site, resulting in a small area where the liquid is applied. Also, the number of puncture positions cannot be increased because of the assembled body.

Because the microneedles used with the mask as described in Patent Document 3 are integrally formed with the beauty ingredients, it is necessary for the microneedles to be left to stand for a long time in order to inject the beauty ingredients dissolved in the microneedles, into the skin. Also, such beauty ingredients that can be integrally formed with the microneedles are limited.

In view of the foregoing, the present invention can reliably achieve a puncture of a plurality of fine needles regardless of deformation of a puncture target, and provide the target with a liquid composition containing any active ingredient without being left to stand for a long time.

### MEANS FOR SOLVING THE PROBLEMS

In order to solve the above problem, one aspect of the present invention provides a fine needle part to be attached to a skin puncture instrument. The fine needle part includes: a plate substrate having a plate shape; a plurality of bases projecting from a surface of the plate substrate, the surface being situated so as to face a target object; and a plurality of needles projecting from top surfaces of the plurality of bases. A height (b) of each of the needles of the plurality of needles is 5 µm ≤ b < 700 µm. A height (a) of each of the bases of the plurality of bases is 200 µm ≤ a < 3,000 µm. A total height (a+b) of the height (b) of each of the needles and the height (a) of each of the bases is from 600 µm through 3,200 µm. The plurality of bases and the plurality of needles are formed of a resin material that does not dissolve. By the target object being punctured by the needles, liquid is applied onto a surface of the target object or to an interior of the target object.

### ADVANTAGEOUS EFFECTS OF THE INVENTION

According to one aspect, the fine needle part can reliably achieve the puncture of the plurality of fine needles even if the puncture target is deformed, and provide the target with the liquid composition containing any active ingredient without being left to stand for a long time.

### BRIEF DESCRIPTION OF THE DRAWINGS

[FIG. 1] FIG. 1 is an overall cross-sectional view of a syringe set in which a fine needle part according to a first embodiment of the present invention is attached to a syringe barrel.
[FIG. 2] FIG. 2 is a view illustrating the syringe set of FIG. 1, with a syringe, a fine needle plate that is the fine needle part, and an attaching frame being separated.
[FIG. 3] FIG. 3 is a cross-sectional perspective view of a fine needle part of a first configuration example of the first embodiment, the fine needle part including a fine needle plate and an attaching frame.
[FIG. 4] FIG. 4 is an enlarged bottom view of the fine needle part of the first configuration example of the first embodiment.
[FIG. 5] FIG. 5 is a cross-sectional view illustrating a puncture and a flow of liquid in the fine needle part of the first embodiment.
[FIG. 6] FIG. 6 is a cross-sectional view of a fine needle part of a second configuration example of the first embodiment.
[FIG. 7] FIG. 7 is a cross-sectional perspective view of a fine needle part of a third configuration example of the first embodiment.
[FIG. 8] FIG. 8 is a cross-sectional perspective view of a fine needle part of a fourth configuration example of the first embodiment.
[FIG. 9] FIG. 9 is a cross-sectional perspective view of a fine needle part of a fifth configuration example of the first embodiment.
[FIG. 10] FIG. 10 is a cross-sectional view illustrating a puncture and a flow of liquid in the fine needle part of FIG. 9.
[FIG. 11] FIG. 11 is a cross-sectional perspective view of a fine needle part according to a first configuration example of a second embodiment.
[FIG. 12] FIG. 12 is a view illustrating a state in which liquid is injected into a target object from the fine needle part of the second embodiment.
[FIG. 13] FIG. 13 is a cross-sectional view of a fine needle part according to a second configuration example of the second embodiment.
[FIG. 14] FIG. 14 is a cross-sectional view of a fine needle part according to a third configuration example of the second embodiment.
[FIG. 15] FIG. 15 is a cross-sectional view of a fine needle part according to a fourth configuration example of the second embodiment.
[FIG. 16] FIG. 16 is a cross-sectional perspective view of a fine needle part according to a third embodiment.
[FIG. 17] FIG. 17 is a view illustrating a state in which liquid is injected into a target object from the fine needle part of the third embodiment.
[FIG. 18] FIG. 18 is an explanatory view of Modified Example 1 of a needle in a lip-equipped two-step needle according to the third embodiment.
[FIG. 19] FIG. 19 is an explanatory view of Modified Example 2 of the needle in the lip-equipped two-step needle according to the third embodiment.
[FIG. 20] FIG. 20 is an explanatory view of Modified Example 3 of the needle in the lip-equipped two-step needle according to the third embodiment.
[FIG. 21] FIG. 21 is a cross-sectional view of a fine needle part according to a first configuration example of a fourth embodiment.
[FIG. 22] FIG. 22 is a bottom view of a fine needle part of the fourth embodiment.
[FIG. 23] FIG. 23 is a cross-sectional view of a fine needle part of a second configuration example of the fourth embodiment.
[FIG. 24] FIG. 24 is a bottom view of a fine needle part of a third configuration example of the fourth embodiment.
[FIG. 25] FIG. 25 is an overall cross-sectional view of a knock-type injection device that is Application Example 2 in which a fine needle part according to an embodiment of the present invention is attached.
[FIG. 26] FIG. 26 is an overall cross-sectional view of an injection device that is Application Example 3 in which the fine needle plate according to the embodiment of the present invention is attached.
[FIG. 27] FIG. 27 is an overall cross-sectional view of a needle applicator that is Application Example 4 in which the fine needle plate according to the embodiment of the present invention is attached.
[FIG. 28] FIG. 28 is an overall cross-sectional view of a head push-in injection device that is Application Example 5 in which the fine needle plate according to the embodiment of the present invention is attached.
[FIG. 29] FIG. 29 is an overall cross-sectional view of a puncture application roller that is Application Example 6 in which the fine needle plate according to the embodiment of the present invention is attached.
[FIG. 30] FIG. 30 is an overall cross-sectional view of a separate liquid-type puncture application roller that is Application Example 6 in which the fine needle plate according to the embodiment of the present invention is attached.
[FIG. 31] FIG. 31 is a table illustrating results of puncture experiments of a fine needle plate of the first configuration example of the first embodiment using bases of different lengths.
[FIG. 32] FIG. 32 is a table illustrating results of puncture experiments of the fine needle plate of the first configuration example of the first embodiment using bases of different front-end diameters.
[FIG. 33] FIG. 33 is a table illustrating results of puncture experiments of the fine needle plate with and without bases, in which a plurality of needles are arranged in rows.
[FIG. 34] FIG. 34 is a table illustrating results of puncture experiments when a plurality of base-equipped needles are arranged in rows, the number of needles is the same, and the pitch thereof is varied.
[FIG. 35] FIG. 35 is a table illustrating results of puncture experiments when a plurality of base-equipped needles are arranged in rows, the number of needles is varied, and the pitch thereof is the same.
[FIG. 36] FIG. 36 is a graph illustrating a ratio of an effective puncture area to a cover area of the fine needle plate of FIG. 35.

### DETAILED DESCRIPTION OF THE INVENTION

Hereinafter, embodiments of the present invention will be described with reference to the drawings. In the following, the same components across the drawings are denoted by the same symbols, and duplicate description may be omitted.

The fine needle part according to the present invention relates to a fine needle part that can be attached to a skin puncture instrument. The fine needle part of the present invention is configured to apply a beauty ingredient in the form of liquid to skin serving as the target object. Also, the fine needle part is mainly for beauty applications and therapeutic applications, and is applied to puncture the skin.

Human skin (skin) is mainly assumed as an example of the target object to which liquid is to be applied by the skin puncture instrument to which the fine needle part of the present invention is attached. The target object can also be, for example, animal skin or the surfaces of a plant stem, trunk, or leaf. Alternatively, the target object may be a tissue, such as skin, organs, or the like removed from test subjects or test animals, or may be an inorganic substance, such as a silicon film, a urethane sheet, or the like. However, these are by no means a limitation.

### <Application Example 1: Syringe set>

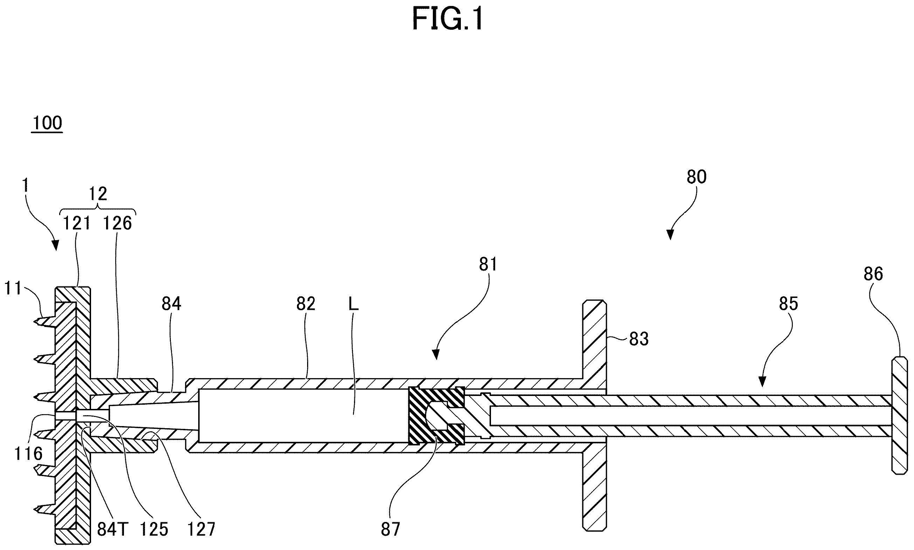

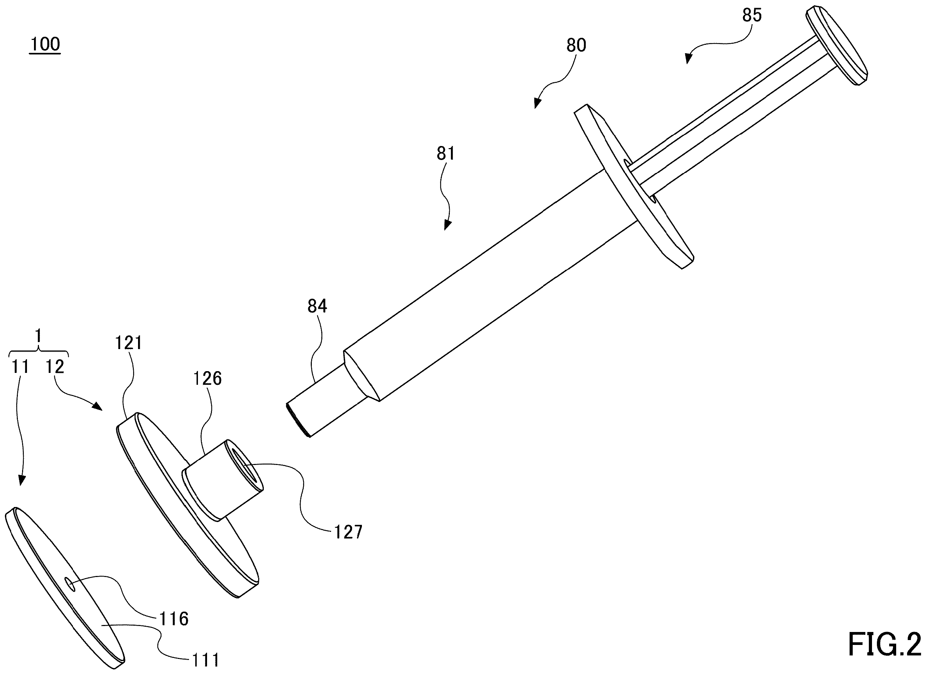

A syringe set in which the fine needle part according to the embodiment of the present invention is connected to a syringe barrel will be described with reference to FIGS. 1 to 4. First, the overall configuration of the syringe set according to the present embodiment will be described with reference to FIGS. 1 and 2.

FIG. 1 is an overall cross-sectional view of the syringe set in which the fine needle plate according to the embodiment of the present invention is attached to the syringe barrel together with the attaching frame. FIG. 2 is a view illustrating a syringe set 100 of FIG. 1, disassembled into a syringe 80, a fine needle plate 11 that is a fine needle part 1, and an attaching frame 12.

As illustrated in FIGS. 1 and 2, the syringe 80 includes a syringe barrel (syringe body, cylinder) 81 and a pusher (plunger) 85. The syringe barrel 81 houses a liquid content L, and the pusher 85 is inserted into the syringe barrel 81. Specifically, the syringe barrel 81 includes an outer barrel 82 housing the content L, a flange 83 provided at the back end of the outer barrel 82, and a front barrel portion 84 that is the front end.

Here, the syringe 80, to which the fine needle plate 11 of the present invention is to be attached via the attaching frame 12, may be a syringe having a volume of, for example, 1 mL, 2.5 mL, 5 mL, or 10 mL. In accordance with a specific manner in which the fine needle plate 11 is used, the syringe may have a larger or smaller volume.

The syringe 80, to which the fine needle part 1 of the present invention is to be attached, is formed of glass, a metal, or a resin. Examples of the resin include polypropylene (PP), polyethylene (PE), acrylonitrile butadiene styrene (ABS), polybutylene terephthalate (PBT), polyacetal (POM), polyethylene terephthalate (PET), polycarbonate (PC), polyether ether ketone (PEEK), cycloolefin polymer (COP), and the like.

In the present embodiment, the fine needle part 1, which is used instead of a typical single hollow syringe needle, is connected to the front barrel portion 84 of the syringe barrel 81.

Specifically, the attaching frame 12 includes a cylindrical attaching barrel 126 that is to be externally fitted to the front barrel portion 84 of the syringe barrel 81. When the fine needle plate 11 is attached to the syringe 80, the fine needle plate 11 is first fitted into the attaching frame 12. Then, the front barrel portion 84 of the syringe barrel 81 is inserted into and engaged with a fitting hole 127 of the attaching barrel 126 of the attaching frame 12. A syringe for use may have a structure of locking: the attaching barrel 126 of the attaching frame 12 to which the fine needle plate 11 is attached; and the front barrel portion 84 of the syringe barrel 81, in order to prevent detachment therebetween. Also, the syringe may have a shape stipulated defined by lure connector standards, such as ISO (International Organization for Standardization), JIS (Japanese Industrial Standards), or the like.

In any of the embodiments, the fine needle part 1 of the present invention includes a two-step needle 112 projecting, at the front end, toward a surface thereof that is to contact the target object.

In response to pushing of a back-end pusher 86 of the syringe 80, the pusher 85 is moved in the outer barrel 82 of the syringe barrel 81, and the content L is discharged from the syringe into the fine needle part 1.

When the content L reaches the interior of the fine needle part 1, the content is delivered to the target object, such as skin, through a flow path 125 and a discharge hole 116. Then, from the front end of the two-step needle 112, the content L enters the target object, such as skin, against which the fine needle plate 11 is pressed.

As illustrated in FIG. 1, a gasket 87 may adhere to the front end of the pusher 85, where the gasket is a thin-plate packing (hermetically sealing element) formed of rubber or elastomer for preventing liquid leakage and contamination. The packing may or may not be provided.

Further, in order to protect a plurality of two-step needles 112 of the fine needle part 1 of the present invention, a cap may be attached to cover the fine needle part 1.

### (Fine needle part according to the first configuration example of the first embodiment)

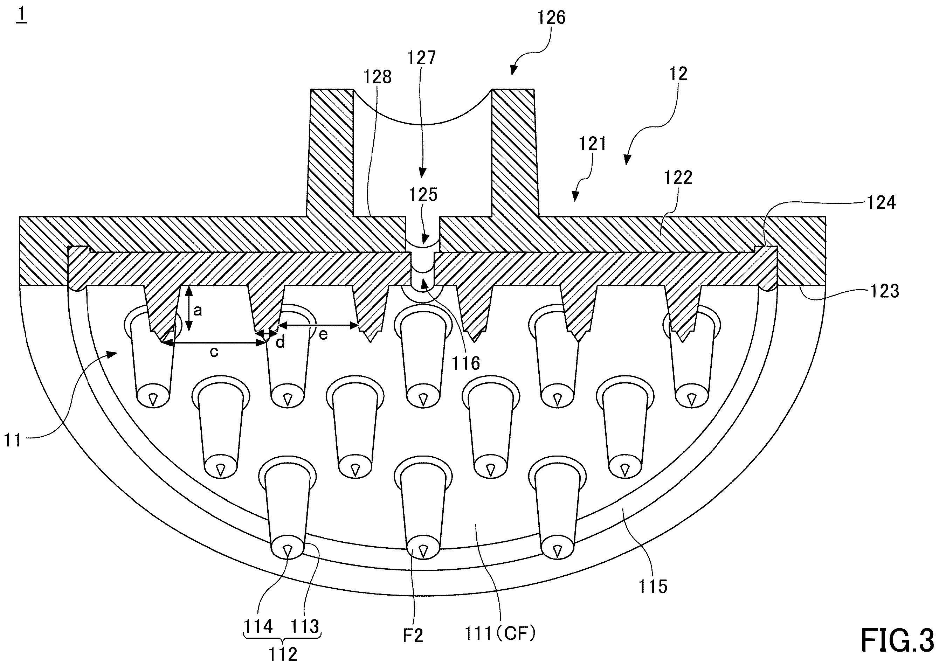

FIG. 3 is a cross-sectional perspective view of the fine needle part of the first configuration example of the first embodiment, the fine needle part including the fine needle plate and the attaching frame.

In the present embodiment, the fine needle part 1 is an assembled body (assembly) of two members, i.e., the fine needle plate 11 and the attaching frame 12.

Meanwhile, the fine needle plate 11 includes: a plate substrate (plate body) 111 having a circular flat plate shape; and the plurality of two-step needles 112 and a lip portion 115 that are provided at the plate substrate 111. The discharge hole 116 is formed at the center of the plate substrate 111. The plate substrate 111 serves as a plate.

In the fine needle plate 11, the plurality of two-step needles 112 project from a front surface CF of the plate substrate 111. Specifically, each of the plurality of two-step needles 112 according to the present embodiment includes a base (base portion) 113 and a needle 114. In the present embodiment, the plate substrate 111 that is a plate base (body), the base 113, and the needle 114 are integrally formed, and the plurality of needles 114 project from the front surfaces of the plurality of bases 113. The two-step needle 112 according to the present embodiment is a two-step solid needle having no opening.

The lip portion 115 is formed on the outer periphery of the front surface (contact surface) CF of the plate substrate 111. In the present embodiment, the lip portion 115 serves as a liquid stopper.

As illustrated in FIG. 3, the height (a) of the base 113 in the fine needle plate 11 is preferably 200 µm ≤ a < 3,000 µm. Also, the height (b) of the needle 114 is preferably 5 µm ≤ b < 600 µm.

In such a case, the total height (a+b) of the height (b) of the needle 114 and the height (a) of the base 113 is preferably from 600 µm through 3,200 µm. Also, a value (b/a) of the ratio of the height (a) of the base 113 to the height (b) of the needle 114 is preferably 0.5 or less. Thus, the height (a) of the base 113 and the height (b) of the needle 114 are set, within the respective recommended ranges thereof, to such dimensions as to satisfy the above total value and the ratio.

As illustrated in FIG. 3, a front-end diameter (d) of the base 113 is preferably 300 µm ≤ d ≤ 600 µm, and the diameter at the root of the needle 114 is set to be smaller than the front-end diameter of the base 113. Also, in order to better contact with skin, the edge of the front surface (top surface) F2 of the base 113 is preferably rounded.

The height of the lip portion 115 from the front surface CF is equal to or shorter (lower) than the length of the two-step needle 112. For example, the height of the lip portion 115 is a height that enables liquid to reach the target object, and is from about 10 µm through about 2,000 µm.

In the present embodiment, the number of the two-step needles 112 may be any number, and is from one through several hundred, with from several two-step needles 112 through about one-hundred two-step needles 112 being more preferable. The plurality of two-step needles 112 may be arranged orderly or provided at random positions. Alternatively, the plurality of two-step needles 112 at the bottom surface may be those formed at one time with one sheet (like a frog) or an assembly of one or more two-step needles-assembled units each including the plurality of two-step needles 112 formed in a row.

In the present embodiment, the needle plate 11 including the two-step needles 112 is preferably formed of a biocompatible material including a biodegradable resin, such as polyglycolic acid, polylactic acid, a polyglycolic acid-polylactic acid copolymer, or the like. However, the needle plate 11 may also be formed of a thermoplastic resin, such as polypropylene (PP), polyethylene (PE), acrylonitrile butadiene styrene (ABS), polybutylene terephthalate (PBT), polyacetal (POM), polyethylene terephthalate (PET), polycarbonate (PC), polyether ether ketone (PEEK), or the like. In addition, biocompatible metal materials, such as stainless steel, cobalt alloys, titanium alloys, and the like, silicone, ceramics, and the like may also be used.

Meanwhile, the attaching frame 12 includes a plate support 121 and the attaching barrel 126.

Specifically, the plate support 121 is a barrel with a top surface, including a round plate 122 to serve as the top surface for housing and an outer barrel 123 to serve as a side wall for housing, and the bottom surface thereof forms a recessed space for housing. At the bottom surface of the round plate 122 to serve as the top surface for housing, the outer periphery to serve as the boundary with the outer barrel 123 is an annular groove 124.

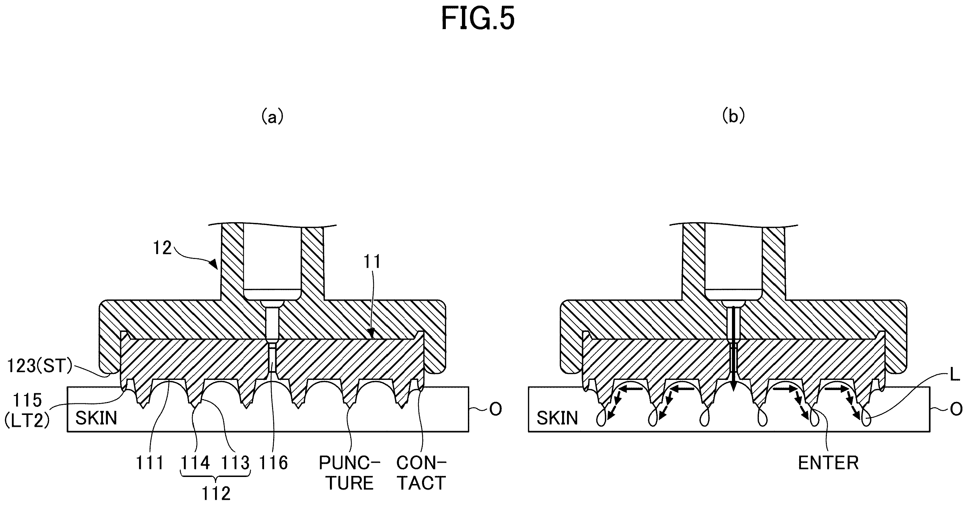

In assembling the fine needle part 1, the outer periphery of the fine needle plate 11 is fitted into the outer barrel 123 of the attaching frame 12. As illustrated in (a) of FIG. 5, in the present embodiment, in a state in which the fine needle plate 11 is attached to the attaching frame 12, a front surface LT of the lip portion 115 in the fine needle part 1 projects outward (lower side in FIG. 5) from a lower end ST of the outer barrel 123. The lower-side direction in FIG. 5 (left side in FIG. 1) is also referred to as an outer side, a front side, or a distal side in an axial direction (with respect to the syringe barrel 81), and the upper-side direction in FIG. 5 (right side in FIG. 1) is also referred to as an inner side, a back side, or a proximal side.

Further, a hole is formed at the center of the plate support 121, and this hole serves as the flow path 125 through which the content passes in the fine needle part.

Meanwhile, the attaching barrel 126 has such a cylindrical shape that one end thereof is open and the other end thereof is coupled to the back surface of the plate support 121. The interior of the barrel serves as the fitting hole 127. The other end of the attaching barrel 126 is a cylindrical perforated bottom surface 128. In FIG. 3, the fitting hole 127 has a straight shape. However, the fitting hole 127 can have any of various typical joint forms, such as a tapered shape, a screw type, a ratchet type, or the like.

Also, the sizes of the flow paths (holes) illustrated in FIG. 3 are in the following order from larger one: the fitting hole 127 > the flow path 125 > the discharge hole 116, as an example. However, the sizes of the holes are not limited to any particular size. With this configuration, a tapered shape of the front barrel portion 84 becomes gradually narrower at the front thereof relative to the attaching barrel 126 when fitting the front barrel portion 84 of the syringe 80 into the attaching barrel 126 in order to attach the fine needle part 1 to the syringe 80. As a result, sealing property is secured by virtue of fitting between the tapers of the side surfaces. When the taper of the front barrel portion 84 has narrowed such that further insertion of the front barrel portion 84 is stopped, as illustrated in FIG. 2, a front end 84T of the front barrel portion 84 may contact the perforated bottom surface 128 for the fitting hole 127 and the flow path 125.

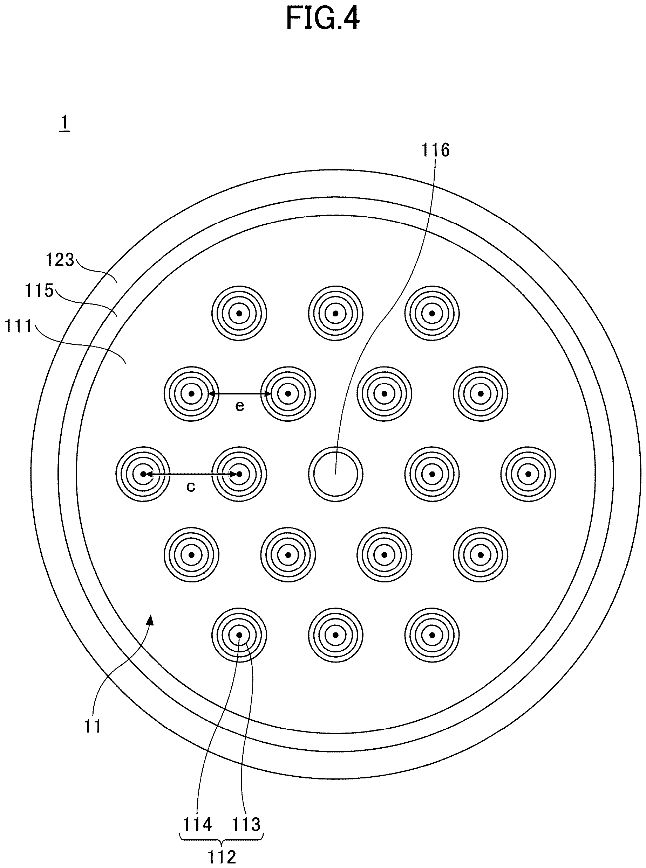

FIG. 4 is a bottom view of the fine needle part of the first configuration example of the first embodiment. According to the fine needle plate 11 of the first embodiment, the number of the two-step needles 112, each being a base-equipped needle in which the base 113 and the needle 114 are formed integrally, may be any number, and is more preferably two or more. When a plurality of base-equipped needles are provided, the base-equipped needles are preferably arranged orderly. FIG. 4 illustrates an example in which the apexes of the needles 114 are arranged in an oblique lattice (triangular arrangement) in a bottom view of the fine needle plate 11. However, the apexes of the needles 114 may be arranged in a lattice (rectangular arrangement).

As illustrated in FIGS. 3 and 4, when the plurality of two-step needles 112, each being a base-equipped needle that is a set of the base and the needle, are provided so as to project from the plate substrate 111, preferably, the bases 113 next to each other are disposed apart from each other, and a pitch (c) of the apexes of the needles 114 is 1 mm ≤ c < 5 mm.

A value ((a+b)/c), i.e., a ratio of the total of the height (a) of the base 113 and the height (b) of the needle 114 to the pitch (c) of the needles 114, is preferably 0.3 or more and 1.1 or less. In this case, an interval (e) between the edges of the front surfaces F2 of the bases 113 is 0.5 mm or more and 4.4 mm or less.

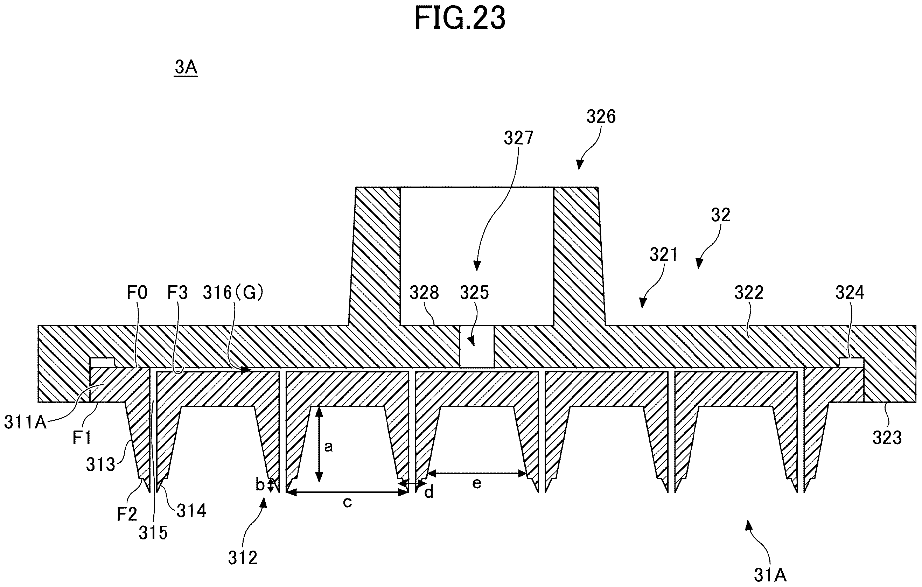

Also, the number of the bases 113 per unit area on the plate substrate 111 is 0.1/cm² or more and 33/cm² or less. The dimensions and effects of these portions will be described in detail together with the results of experimental examples illustrated in FIGS. 23 to 28.

FIG. 5 is a cross-sectional view illustrating a puncture and a flow of liquid in the fine needle part of the first embodiment with respect to a target object O.

By pushing the syringe barrel 81 of the syringe 80 to the target object O, such as skin, the target object O is punctured by the plurality of needles 114 while being pushed and stretched by the bases 113 of the plurality of two-step needles 112 (see (a) of FIG. 5). At this time, the lip portion 115 of the outer periphery of the contact surface CF of the fine needle part 1 contacts the target object O.

Subsequently, in response to pushing of the pusher 85 of the syringe 80, the liquid L reserved in the syringe 80 flows into the fine needle part 1, and the liquid spreads in a space defined by the target object O, the lip portion 115, and the front surface CF of the plate substrate 111 and enters the skin through holes formed by the plurality of two-step needles 112. In other words, the liquid penetrates through the gaps between the skin and the front surfaces of the bases 113 of the two-step needles 112 (see (b) of FIG. 5).

After injection, for a predetermined time (less than about one second through about 30 minutes, preferably about several seconds through about several minutes), the fine needle part 1 is maintained in a state of being pressed against the target object O via the syringe.

After the predetermined time has passed, the syringe 80 is taken away from the target object O, thereby withdrawing the needles 114 of the two-step needles 112 from the target object O.

As described above, in the present invention, the liquid having a beauty effect on skin as the target object O spreads on the skin radially inward of the lip portion 33. At the same time, the base 113 of the two-step needle 112 pushes and stretches the skin so as to be readily punctured, and the liquid penetrates into the target object O through the holes formed in the stretched skin by the puncture of the needles 114. Therefore, it is possible for the active ingredients of the liquid to penetrate into the target object O both from the exterior thereof (the surface side) and from the interior thereof (the holes formed by the puncture). For example, when the target object O is skin, beauty ingredients of the liquid can penetrate into the skin from both of the exterior and the interior of the skin.

### <Second configuration example of the fine needle part of the first embodiment>

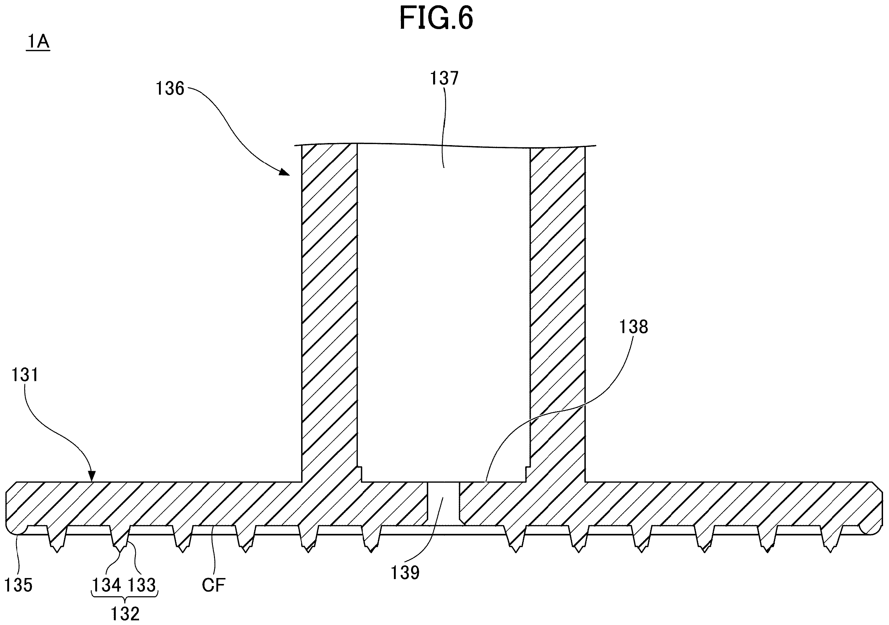

Next, a second configuration example of the fine needle part will be described with reference to FIG. 6. FIG. 6 is a cross-sectional view of a fine needle part 1A of the second configuration example of the first embodiment.

The above-described fine needle part 1 is an assembled structure (assembly structure) of two members, i.e., the attaching frame 12 and the fine needle plate 11. However, the fine needle part may be a single portion in which a puncturing function is integrated with a function of attaching to the front barrel portion of the syringe.

In the present embodiment, the fine needle part 1A includes a fine needle plate 131 and an attaching barrel 136 in an integrated manner.

The fine needle part 1A of an integrated type according to the present embodiment is preferably formed of a biocompatible material including a biodegradable resin, such as polyglycolic acid, polylactic acid, a polyglycolic acid-polylactic acid copolymer, or the like. However, the fine needle part 1A may also be formed of a thermoplastic resin, such as polypropylene (PP), polyethylene (PE), acrylonitrile butadiene styrene (ABS), polybutylene terephthalate (PBT), polyacetal (POM), polyethylene terephthalate (PET), polycarbonate (PC), polyether ether ketone (PEEK), or the like. In addition, biocompatible metal materials, such as stainless steel, cobalt alloys, titanium alloys, and the like, silicone, ceramics, and the like may also be used.

The fine needle plate 131, which is a plate substrate having a circular flat plate shape, is provided with a lip portion 135, and a plurality of two-step needles 132 each having a base 133 and a needle 134. A discharge hole 139 is formed at the center of the fine needle plate 131.

The height of the lip portion 135 from the front surface CF is lower (shorter) than the height of the two-step needle 132.

The attaching barrel 136 includes a fitting hole 137. With this configuration, a portion between the radially inner peripheral surface of the fitting hole 137 and the radially outer surface of the discharge hole 139 is a cylindrical perforated bottom surface 138. In a state in which the attaching barrel 136 is engaged with the front barrel portion 84 of the syringe 80, the content L flowing from the flow path of the front barrel portion 84 hits and passes through the perforated bottom surface 138. In order for the liquid to move smoothly, the perforated bottom surface 138 may have a shape tapered or curved toward the discharge hole 139 that is a through-hole. The perforated bottom surface 138 may have a screw structure or a ratchet structure.

In the present embodiment, by providing the two-step needles 132 and by providing the lip portion 135 integrally with the bottom surface of the fine needle plate 131, the liquid having an effect on the target object spreads on the skin radially inward of the lip portion 135. At the same time, the base 133 stretches the skin so as to be readily punctured, and the liquid penetrates into the target object through the holes formed in the stretched skin by the needles 134. Therefore, active ingredients of the liquid can penetrate into the target object from both of the exterior and the interior of the target object. For example, when the target object is skin, beauty ingredients of the liquid can penetrate into the skin from both of the exterior and the interior of the skin.

### <Third configuration example of the fine needle part of the first embodiment>

The above describes an example in which the needle plate has one discharge hole at the center thereof. However, the number of the discharge holes may be two or more, and the position of the discharge hole may or may not be at the center.

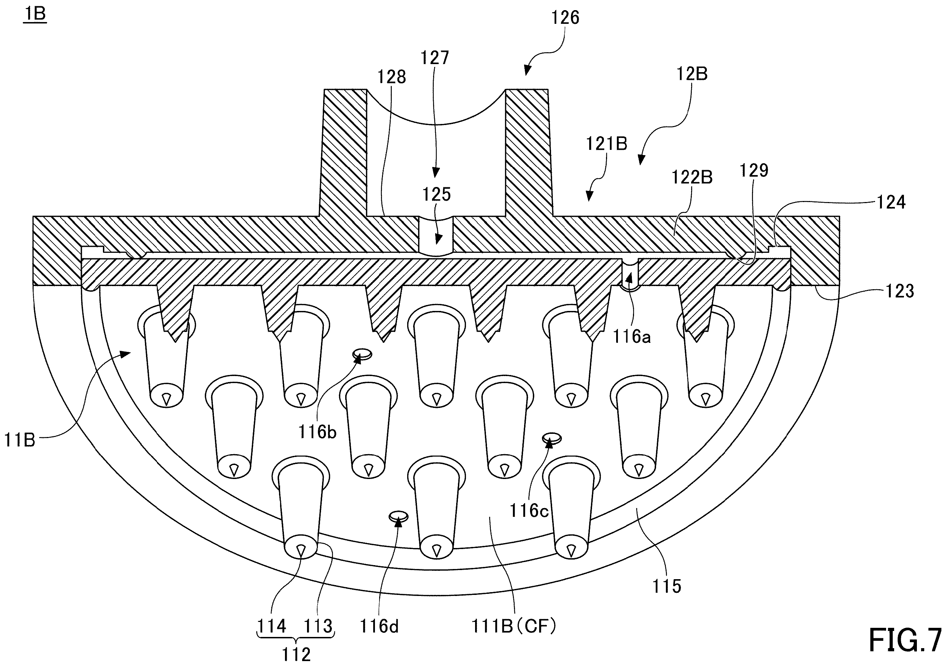

FIG. 7 is a cross-sectional perspective view of the fine needle part 1A of the third configuration example of the first embodiment. In the present configuration example, in a fine needle plate 11B, the positions of vertically penetrating discharge holes 116a to 116d are not at the center, and a plurality of discharge holes are provided. In order for the liquid from the flow path 125 in the attaching barrel 126 to be moved to the discharge holes 116 to 116d apart from the center, a gap for the liquid to pass through is provided between an attaching frame 12B and a plate substrate 111B of the fine needle plate 11B.

In order to achieve the gap, in the present embodiment, the front surface of a round plate 122B of a plate support 121B of the attaching frame 12B is provided with an annular projection 129 at a certain distance from the center. When the fine needle plate 11B is attached to the attaching frame 12B, the front end of the annular projection 129 contacts a back surface BF of the plate substrate 111B, whereby a region enclosed by the annular projection 129 and the back surface BF of the plate substrate 111B becomes a partitioned gap space. When the liquid enters the gap space from the flow path 125 of the attaching frame 12B, an accumulation of liquid is formed. The liquid can flow from the accumulation of liquid into the discharge holes 116a to 116d.

Therefore, in the present embodiment, the positions of the discharge holes 116a to 116d, provided in the plate substrate 111B to serve as a plate, are set so as to be closer to the center than is the position of the annular projection 129 of the attaching frame 12B.

According to the fine needle part 1B of the present embodiment, the liquid from the attaching barrel 126 flows through the flow path 125 at the center of the perforated bottom surface 128 and spreads in the gap space on the top surface of the plate substrate 111B as an accumulation of liquid, followed by entering the plurality of discharge holes 116a to 116d.

When the liquid that has entered the plurality of discharge holes 116a to 116d is expelled outside the front surface CF of the plate substrate 111B, the liquid spreads on the target object toward the inner periphery of the lip portion 115. At the same time, the base 113 of the two-step needle 112 pushes and stretches the skin so as to be readily punctured, and the liquid penetrates into the target object O through the holes formed in the stretched skin by the needles 114. Therefore, active ingredients of the liquid can penetrate into the target object from both of the exterior and the interior of the target object.

In the present embodiment, the plurality of discharge holes 116a to 116d, apart from the center, and the gap space form a two-step accumulation of liquid at the back surface and the front surface of the plate substrate 111B. This makes the liquid readily spread radially outward. Therefore, even if the liquid is applied to the target object on which the liquid does not readily spread from the center to the lip portion 115 as the outer peripheral edge due to roughness of the surface or projections on the surface of the target object, it is possible to sufficiently spread the liquid radially outward from the center because there are the discharging holes 116a to 116d at the positions located partway from the center to the lip portion 115.

The number of the discharge holes 116a to 116d is two or more. In the present configuration, increasing the number of discharge holes can more enhance the effect of spreading radially outward from the center.

Moreover, in the present configuration, in order for the liquid to be more efficiently moved radially outward from the center in the gap space between the plate substrate 111B and the round plate 122B of the attaching frame 12B, by using as a flow path a portion having the shortest path through which the liquid flows from the center to the discharge holes 116a to 116d, a band-shaped recess may be formed at the back surface BF of the plate substrate 111B.

### <Fourth configuration example of the fine needle part of the first embodiment>

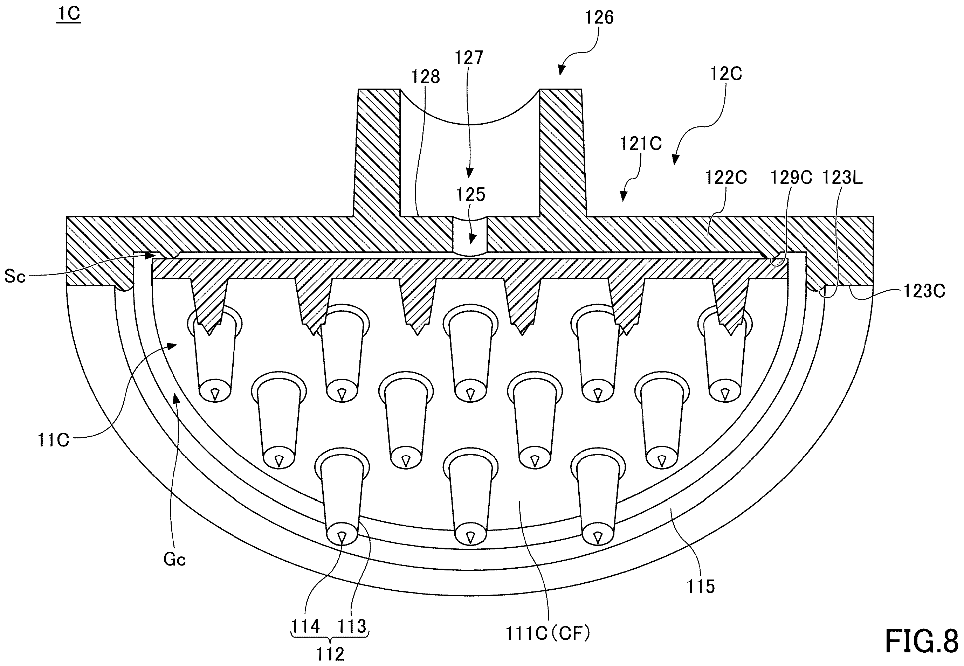

FIG. 8 is a cross-sectional perspective view of a fine needle part of the fourth configuration example of the first embodiment. In the present configuration example, a fine needle plate 11C is not provided with a vertically penetrating discharge hole, and the fine needle plate 11C is smaller than the inner periphery of the outer barrel 123C of an attaching frame 12C. The radially inner portion of the outer barrel 123C of the attaching frame 12C is a lip portion 123L projecting toward the front side. Therefore, a groove between the radially outer periphery of a plate substrate 111C and the lip portion 123L of the attaching frame 12C is an annular flow path Gc through which liquid can flow annularly.

Similar to the third configuration example, the liquid from the flow path 125 in the attaching barrel 126 has to be moved to the annular flow path Gc apart from the center in the present configuration example. Thus, by providing the annular projection 129, a gap space Sc for the liquid to pass through is provided between the attaching frame 12C and the plate substrate 111C of the fine needle plate 11C. The configuration of the gap is similar to that in the third configuration example.

According to the fine needle part 1C of the present embodiment, the liquid from the attaching barrel 126 flows through the flow path 125 at the center of the perforated bottom surface 128 and spreads in the gap space Sc on the top surface of the plate substrate 111C as an accumulation of liquid, followed by entering the outer annular gap Gc.

When the liquid that has entered the radially outer annular gap Gc reaches the target object, the liquid spreads toward the center of the circle from the lip portion 123L provided in the attaching frame 12C. At the same time, the base 113 of the two-step needle 112 pushes and stretches the skin so as to be readily punctured, and the liquid penetrates into the target object O through the holes formed in the stretched skin by the puncture of the needles 114. Therefore, active ingredients of the liquid can penetrate into the target object from both of the exterior and the interior of the target object.

### <Fifth configuration example of the fine needle part of the first embodiment>

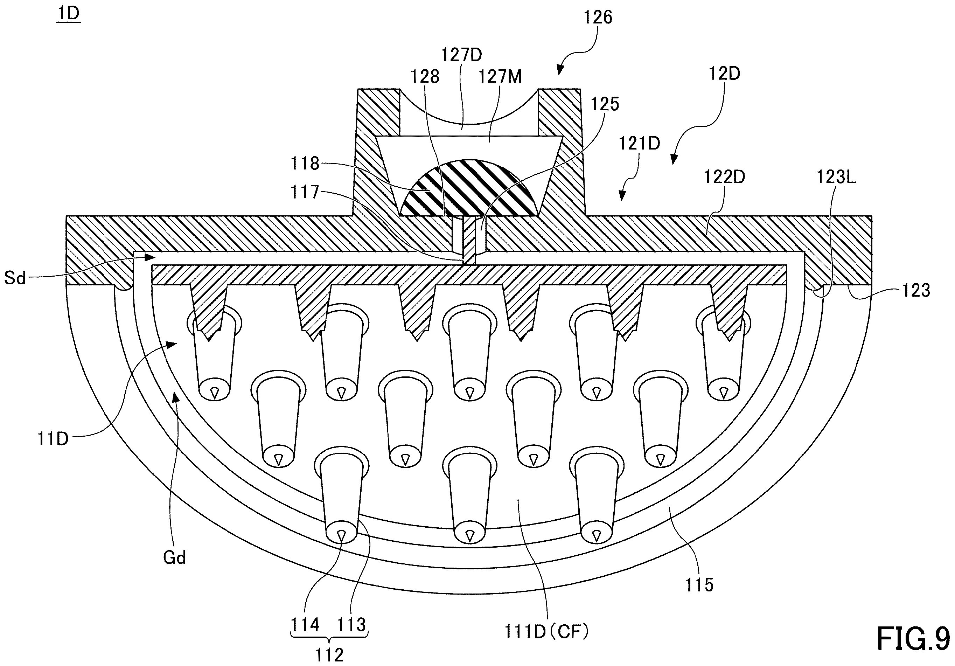

FIG. 9 is a cross-sectional perspective view of a fine needle part 1D of the fifth configuration example of the first embodiment.

In the present configuration example, a shaft 117 inserted into a hole 127D is provided on the back surface facing the needle plate 11, and a sealing valve 118 is provided at the free end of the shaft. Further, a slightly large movable space 127M, in which the sealing valve 118 is movable, is formed at the lower end of the fitting hole 127D in the barrel of the attaching barrel 126 of an attaching frame 12D.

With this configuration, in the present configuration example, a fine needle plate 11D is configured to be raised and lowered through pressing against the attaching frame 12D. In the non-use state in which no force is applied and the plate is directed downward, the back surface of the fine needle plate 11D is apart from the front surface of a round plate 122D of the attaching frame 12D.

With this configuration, in addition to passage of the liquid is achieved through pushing of the pusher as described above, passage of the liquid is achieved by the sealing valve 118 when the fine needle plate is contacted with the skin and pushed upward. Alternatively, instead of pushing of the pusher, passage or non-passage of the liquid may be set only by the sealing valve 118.

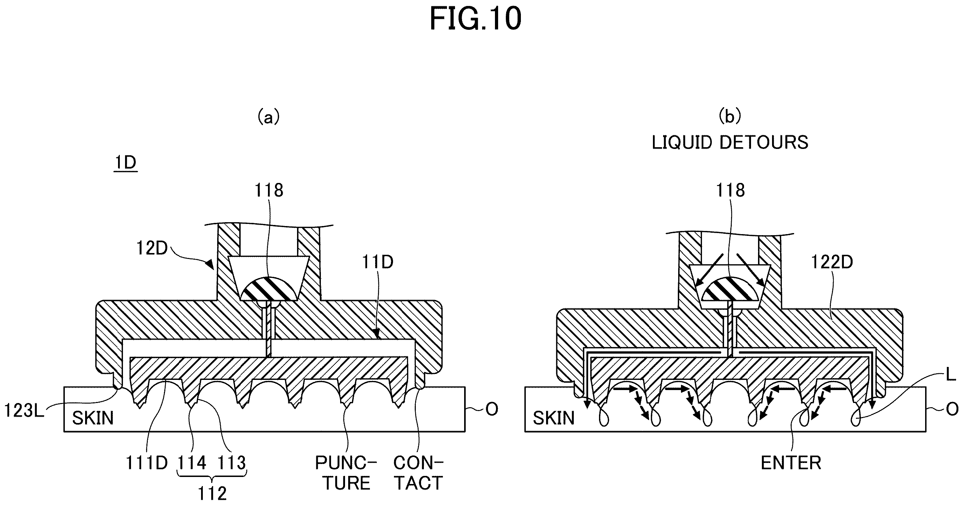

Details of how the present configuration example works will be described with reference to FIG. 10. FIG. 10 is a view illustrating how the fine needle part 1 according to the fifth configuration example works on the target object O.

By pushing the syringe barrel 81 of the syringe 80 to the target object O, such as skin, the skin is deformed and stretched by the base 113 of the two-step needle 112, thereby facilitating insertion of the needles, and the target object O is punctured by the needles 114 (see (a) of FIG. 10). At the same time, the two-step needles 112 on the front side of the fine needle part 1D contact the skin, and thus the fine needle plate 11D is pushed back by the skin.

Subsequently, in response to pushing of the fine needle plate 11D in accordance with pushing of the pusher 85 of the syringe 80, the back surface of the plate substrate 111D becomes closer to the front surface of the round plate 122D of the attaching frame 12D, thereby releasing the sealing valve 118 in an attaching barrel 26D. Thus, the liquid L reserved in the syringe 80 flows into the fine needle part 1D and flows through a gap space Sd into an annular flow path Gd. The liquid spreads in the space formed between the target object O, the lip portion 123L, and the front surface CF of the plate substrate 111D. At the same time, the base 113 of the two-step needle 112 pushes and stretches the skin so as to be readily punctured, and the liquid penetrates into the skin through the holes formed in the stretched skin by the needles 114. That is, the liquid penetrates, at least, through the gap between the front surface of the base 113 and the skin (see (b) of FIG. 10).

### <Fine needle part of the first configuration example of the second embodiment>

In the above-described first embodiment, the base and the needle are formed integrally with the fine needle plate located on the outermost (front) side of the fine needle part. However, the two-step needle of the fine needle part of the present invention may be such that the base and the needle are formed at a separate plate.

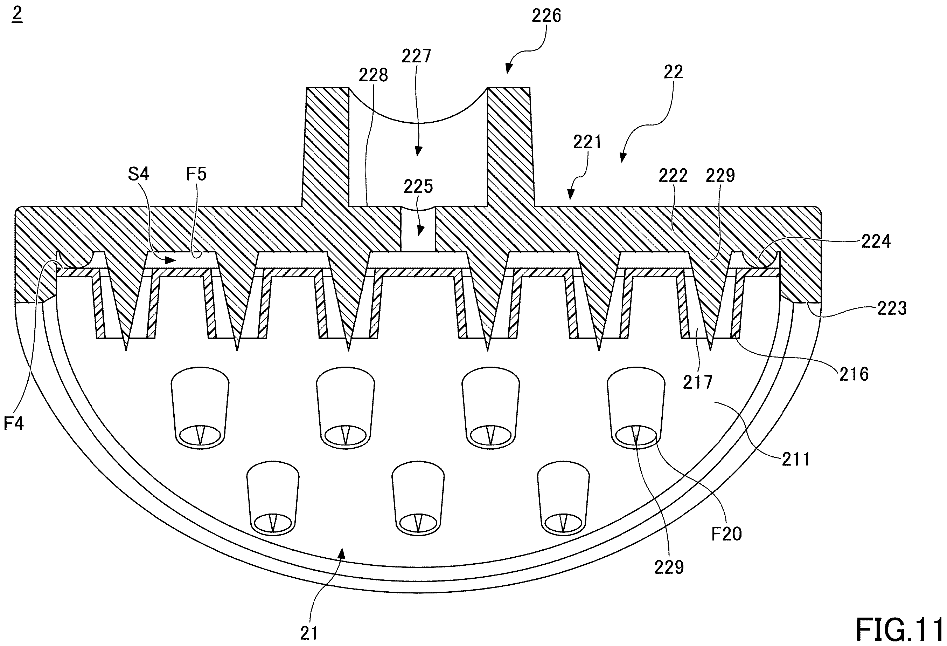

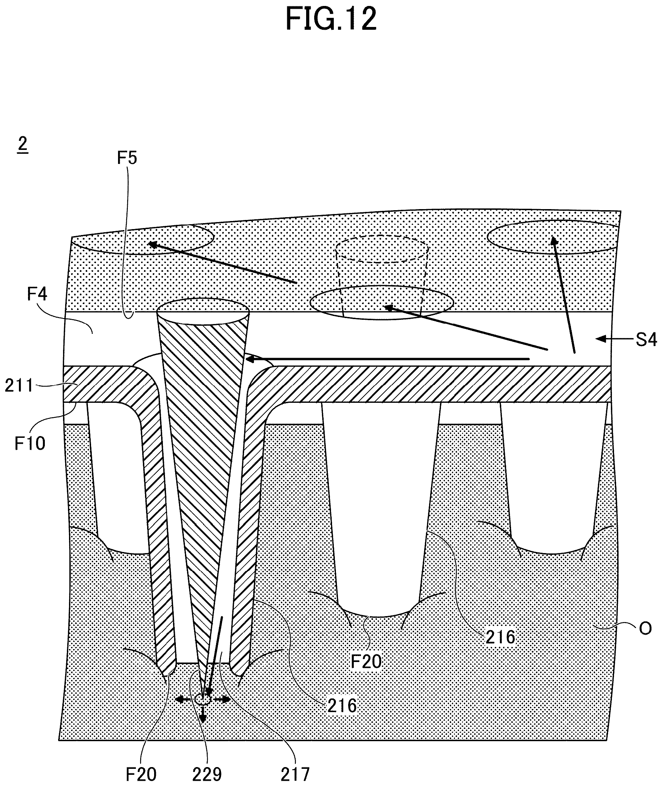

A second embodiment in which the base and the needle are formed at a separate plate in the fine needle part will be described with reference to FIGS. 11 and 12. FIG. 11 is a cross-sectional enlarged perspective view of a fine needle part 2 according to a first configuration example of the second embodiment of the present invention. FIG. 12 is a view illustrating a state in which liquid is injected into a target object from the fine needle part 2 of the second embodiment of the present invention.

Similar to the above, an attaching frame 22 of the fine needle part 2 of the present embodiment includes a plate support 221 and an attaching barrel 226. The plate support 221 is a top surface-including barrel that includes: a round plate-shaped flow path fine needle plate 222 to serve as a top surface for housing; and an outer barrel 223 to serve as a side wall for housing. The bottom surface thereof is a recessed housing space. Also, an annular projection 224 is provided at the outer periphery of the flow path fine needle plate 222 to serve as the top surface for housing.

However, according to the attaching frame 22 in the present configuration, a needle-like projection (also referred to as a solid needle) 229 is provided at the back surface of the flow path fine needle plate 222 in which a flow path hole 225 is formed. Also, an outer (front) contact plate 21 is provided, instead of the base, with a barrel 216 having a space 217 through which the needle-like projection 229 can be inserted. Therefore, the fine needle part 2 of the present configuration example includes: the attaching frame 22 including the flow path fine needle plate 222 provided with a flow path hole 25 and the needle-like projection 229; and the contact plate 21 including the barrel 216.

As illustrated in FIG. 11, according to the attaching frame 22, one or more conical needle-like projections 29 project (hang down) from an outer surface (lower surface) F5 of the flow path fine needle plate 222 including the flow path hole 25 formed at the center thereof.

Meanwhile, the contact plate 21 includes one or more barrels 216 projecting (hanging down) from a contact surface (first surface, bottom surface) F10 of an outer plate substrate 211. The contact surface F10 faces the target object, and the outer plate substrate 211 is disposed to face the outer side (front end) of the flow path fine needle plate 222.

At this time, the barrels 216 are arranged in the same number as that of the needle-like projections 229 projecting from the flow path fine needle plate 222 such that the needle-like projections 229 are positioned radially inward of the barrels 216 of the contact plate 21. In the present embodiment, the barrel 216 serves as the base, and a portion of the needle-like projection 229 projecting from the barrel 216 serves as the needle. Thereby, the barrel 216 and the needle-like projection 229 form the two-step needle.

According to the fine needle part 2 of the present configuration example, as illustrated in FIG. 12, the liquid passing through the flow path hole 25 spreads as an accumulation of liquid in a space S4 formed by the lower surface F5 of the flow path fine needle plate 222 and an upper surface F4 of the outer plate substrate 211. Subsequently, in the space 217 of the barrel 216, the liquid enters and flows into the cylindrical gap between the outer surface of the needle-like projection 229 and the inner peripheral surface of the barrel 216, and the liquid enters the interior of the target object O through the hole formed by the needle-like projection 229.

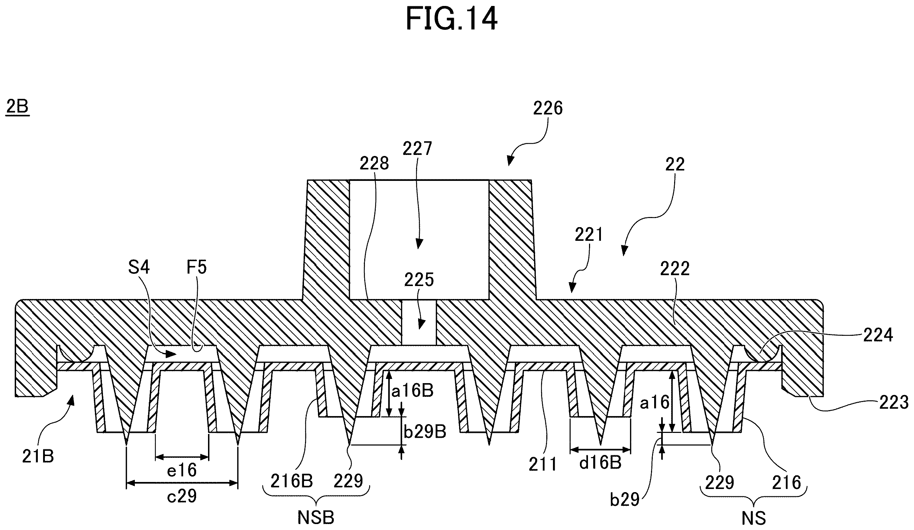

FIGS. 11 and 12 illustrate an example in which the upper surface F4 of the outer plate substrate 211 and the lower surface F5 of the flow path fine needle plate 222 are both flat surfaces. However, as illustrated in FIG. 14 described below, a communication path may be formed by providing radial grooves at the upper surface of the outer plate substrate 211. Alternatively, the communication path may be formed by contacting the upper surface F4 of the outer plate substrate 211 with the lower surface F5 of the flow path fine needle plate 222 and by providing radial grooves at the lower surface F5 of the flow path fine needle plate 222.

Alternatively, the communication path may be formed by providing a filler in a gap formed by disposing, in a non-contact manner, the upper surface F4 of the outer plate substrate 211 in proximity to the entire area of the lower surface F5 of the flow path fine needle plate 222. Specifically, the filler may be provided in a region other than the region that is the shortest path for the liquid to flow from the center to the space 217 of the barrel 216.

With this configuration, as illustrated in FIG. 12, the liquid spreads on the target object O on the inner peripheral side of a front edge F20 of the barrel 216, and at the same time penetrates into the interior of the target object O through the holes formed by the needle-like projections 229. Therefore, active ingredients of the liquid can penetrate into the target object O from both of the exterior and the interior of the target object O.

In the present embodiment, as illustrated in FIG. 12, the liquid having a beneficial effect on the target object is spread over the skin radially inward of the front edge F20 of the barrel 216 in a state in which the skin is deformed and stretched by the barrel 216. At the same time, the liquid penetrates into the target object through the holes formed by the needle-like projections 229 that are solid needles. Thereby, active ingredients of the liquid (e.g., beauty ingredients) can penetrate into the target object from both of the exterior of the surface of the target object and the interior of the target object. For example, when the second embodiment is applied, after unsealing the syringe 80 or pouring the liquid into the syringe barrel 81 immediately before use, the small fine needle part is attached to the syringe, followed by injection, thereby applying the beauty ingredients in fresh liquid onto the skin and into the skin.

At this time, as illustrated in FIG. 12, the gap enclosed by the target object and the front edge F20 of the barrel 216 is small, and thus the liquid reaches the entire region enclosed by the front edge F20 of the barrel 216. Specifically, in the space enclosed by the skin and the front edge F20 of the barrel 216, the liquid may first accumulate in a lower space thereof, but eventually spreads upward as the space is filled with the liquid. In this manner, by preventing the liquid from leaking from the gap by the front edge F20 of the barrel 216, the active ingredients can be retained on the surface of the target object and in the interior of the target object. Thereby, even if the syringe 80 is oriented upward or sideways, the active ingredients of the liquid can be provided on and in the surface of the target object by the fine needle part 2.

In the present embodiment, as illustrated in FIGS. 11 and 12, the front end of the needle-like projection 229 is positioned on an outer side (a lower side, a distal side) of the front edge F20 of the barrel 216, and thus the needle projection 229 and the barrel 216 form the two-step needle. Therefore, in the present configuration, preferably, the height of the barrel 216 is 200 µm or more and less than 3,000 µm, and the height of the needle-like projection 229 outward of the contact surface F10 is from 600 µm through 3,200 µm.

Also, in the present configuration, the outer diameter of the front edge F20 of the barrel 216 corresponding to the base of the two-step needle is preferably larger than 200 µm and 600 µm or less.

As illustrated in FIGS. 11 and 12, the plurality of barrels 216 and the plurality of needle-like projections 229 are provided apart from each other. In an assembled state, the pitch between the apexes of the needle-like projections 229 positioned in the barrel 216 is preferably 1 mm or more and less than 5 mm. In this case, the interval between the edges of the top surfaces of the barrels 216 (the outer peripheries of the front edges F20) is preferably 0.5 mm or more and 4.4 mm or less.

Also, the number of barrels 216 per unit area on the outer plate substrate 211 is preferably 0.1/cm² or more and 33/cm² or less.

As illustrated in FIG. 11, the example of the fine needle part according to the present embodiment is formed of two members, i.e., the attaching frame 22 including the flow path fine needle plate (flow path plate) 222; and the contact plate 21. However, the flow path fine needle plate may or may not be integrated with the attaching frame. For example, the flow path fine needle plate 222 may be attached so as to face the contact plate 21 by a separate member configured to retain two plates at a predetermined distance (e.g., a retaining member configured to retain an outer periphery, or a bonding member configured to bond two plates with a predetermined thickness).

### <Second configuration example of the second embodiment>

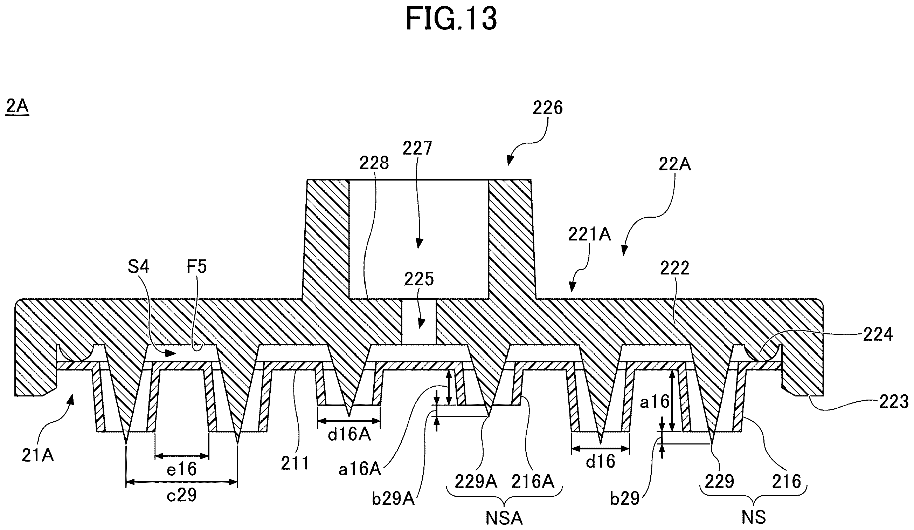

The second embodiment illustrated above in FIG. 11 can take various modified examples in which the height is different in some portions. FIG. 13 is a cross-sectional view of a fine needle part 2A according to a second configuration example of the second embodiment.

As illustrated in FIG. 13, the present configuration example includes a plurality of needle sets NS of the needle-like projections 229 and the barrels 216 that project on the outer plate substrate 211 of the contact plate 21A. There are needle sets NSA that are different from the plurality of needle sets NS in terms of both the height of the needle-like projections 229 and the height of the barrels 216, i.e., the lengths of projection from the outer plate substrate 211. That is, the length of some of the needle sets NS, i.e., the entire two-step needles, is varied.

In the present configuration example, it is possible to achieve punctures of various depths with a single puncture. When changing the length of the needle set, for example, it is preferable to set the above length to be shorter near the center in the radial direction and to be longer near the outer periphery. When changing the length of some of the needle sets NS, it is preferable that the change in the length of the needle set NS of interest be ±15% or less with respect to the other needle sets.

### <Third configuration example of the second embodiment>

FIG. 14 is a cross-sectional view of a fine needle part 2B according to a third configuration example of the second embodiment. As illustrated in FIG. 14, the present configuration example is such that the plurality of barrels 216 of the plurality of needle sets NS include some of the barrels 216 that are different in height of projection from the outer plate substrate 211 without changing the height of projection of the needle-like projections 229 from the flow path fine needle plate 222. That is, in the two-step needles, some of the barrels 216 to serve as the bases are varied in height.

In the present configuration example, it is possible to achieve punctures of various depths with a single puncture. When changing the length of the barrel of the needle set, for example, it is preferable to set the above length to be shorter near the center in the radial direction and to be longer near the outer periphery. When changing the length of some of the barrels of the needle sets NS, it is preferable that the change in the length of the barrel of the needle set NS of interest be ±15% or less with respect to the barrels of the other needle sets.

### <Fourth configuration example of the second embodiment>

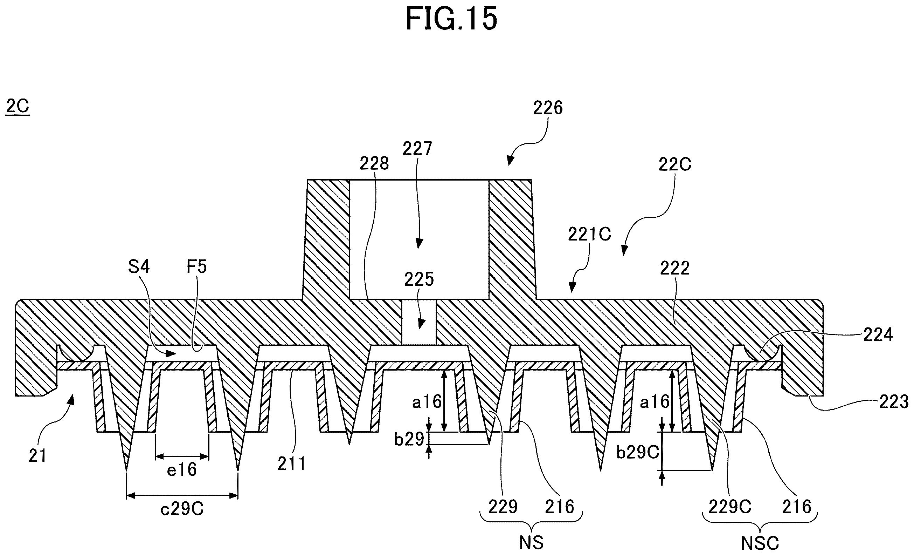

FIG. 15 is a cross-sectional view of a fine needle part 2C according to a fourth configuration example of the second embodiment. As illustrated in FIG. 15, the present configuration example is such that the plurality of needle-like projections 229 of the plurality of needle sets include some of the needle-like projections 229 that are different in height of projection from the flow path fine needle plate 222 without changing the height of projection of the barrels 216 from the outer plate substrate 211. That is, in the two-step needles, the needles projecting from the bases of the same height are changed in height.

According to the present configuration example, it is possible to achieve punctures of various depths with a single puncture. When changing the length of the barrel of the needle set, for example, it is preferable to set the above length to be shorter near the center in the radial direction and to be longer near the outer periphery. When changing the length of some of the needle-like projections of the needle sets NS, it is preferable that the change in the length of the needle-like projection of the needle set NS of interest be ±15% or less with respect to the needle-like projections of the other needle sets.

In the configuration examples illustrated in FIGS. 13 to 15, the length of some of the needle sets in FIG. 13, the length of some of the barrels in FIG. 14, and the length of some of the needle-like projections in FIG. 15 are changed. Heights a16, a16A, a16B, and a16C from the outer plate substrate 211 to the front surfaces of the barrels 216, 216A, and 216B are all 200 µm or more and less than 3,000 µm. Also, heights of projection (height differences) b29, b29A, b29B, and b29C of the needle-like projections 229, 229A, and 229C from the front surfaces of the barrels 216 (216A, 216B) are less than the heights a16, a16A, a16B, and a16C of the barrels 216, and are preferably from 5 µm through 700 µm, and more preferably from 10 um through 200 µm.

In any of the configuration examples, the plurality of sets NS are provided apart from each other, and a pitch c29 between the apexes of the needle-like projections 229 (229A, 229C) is preferably 1 mm ≤ c29 < 5 mm.

In the present configuration, outer diameters d16 (d16A, d16B) of the front surfaces of the barrels 216 (216A, 216B) are preferably greater than 200 um and 600 µm or less.

Considering deformation of the skin, a value ((a16+b29)/c29), i.e., a ratio of the total of the height a16 of the barrel 216 and the height difference b29 of the needle-like projection 229 to the pitch c29 of the needle-like projections 229, is preferably 0.3 or more and 1.1 or less. In this case, preferably, an interval e16 between the outer peripheries of the barrels 216 is less than the pitch c29 and is 0.5 mm or more and 4.4 mm or less.

When dimensions fall within the above-described ranges, even if the length of the needle set, the length of the barrel, or the length of the needle is partially changed, the liquid having a beneficial effect on the target object is spread over the skin radially inward of the front edge of the barrel 216 in a state in which the skin is deformed and stretched by the barrel 216. At the same time, the liquid penetrates into the target object through the holes formed by the needle-like projections 229 that are solid needles.

However, in accordance with the state of the target object (e.g., when the skin is hard), for example, when the height difference b29A in the needle-like projection 229A of the needle set NSA in FIG. 13 is shorter than the other height differences, the target object cannot be punctured by this needle-like projection. In this case, the liquid having a beneficial effect on the target object penetrates into the target object only from the exterior of the target object while spreading the liquid over the skin radially inward of the front edge of the barrel 216 in a state in which the skin is deformed and stretched.

### <Third embodiment>

According to the fine needle part 1 according to the above-described first embodiment, the two-step needles, the bases, and the needles are integrally formed with the outermost plate substrate 111 of the fine needle plate 11, and the discharge holes 116 are formed in the plate substrate 111 at sites different from those of the two-step needles. However, in the fine needle part of the present invention, holes through which the liquid passes may be formed in the bases.

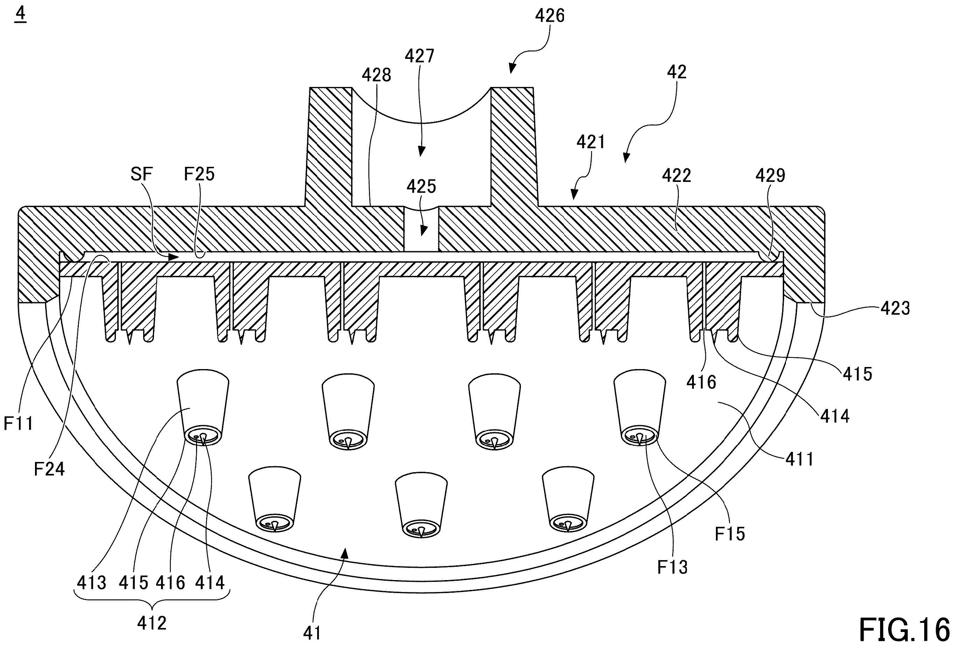

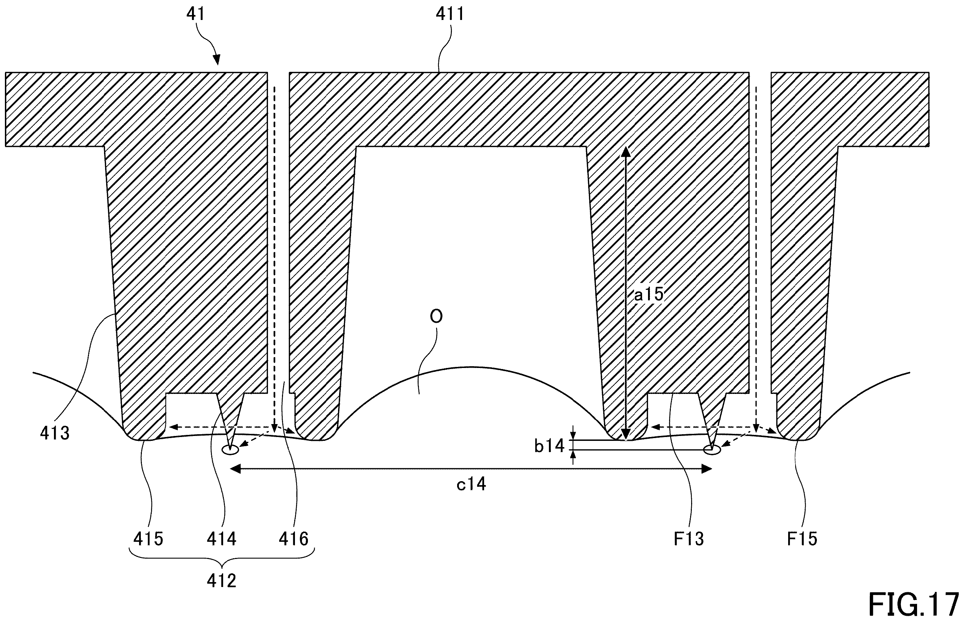

A third embodiment in which holes are formed in the bases will be described with reference to FIGS. 16 and 17. FIG. 16 is a cross-sectional perspective view of a fine needle part 4 according to the third embodiment of the present invention. FIG. 17 is a view illustrating a state in which liquid is injected into the target object from the fine needle part 4 of the third embodiment.

The fine needle part 4 of the present embodiment includes a fine needle plate 41 and an attaching frame 42. The attaching frame 42 has the same configuration as that of the attaching frame 12B illustrated in FIG. 7.

In the fine needle plate 41, a plurality of lip-equipped two-step needles 412 project from a front surface F11 of a plate substrate 411. Each of the lip-equipped two-step needles 412 includes a base 413 and a needle 414 that are integrally formed with the plate substrate 411.

The outer periphery of the front surface (top surface) of the base 413 is longer than a front recessed surface F13 that is the center front surface in the radial direction, and is formed in a lip shape as an individual lip 415 so as to be positioned outward (distal side) of the radial center. That is, the radial center of the front surface of the base 413 is recessed toward the proximal side.

As illustrated in FIGS. 16 and 17, one or more discharge holes 416 through which fluid passes are formed in the needle 414-free portions of the front recessed surfaces F13 of the bases 413 so as to vertically penetrate the plate substrate 411 and the bases 413.

Also, in each of the lip-equipped two-step needles 412, the length of projection of the needle 414 from the front recessed surface F13 is longer than the length of projection of the individual lip 415, i.e., the outer periphery, from the front recessed surface F13. Therefore, the individual lip 415 serves as a liquid stopper in the lip-equipped two-step needle 412.

Also, a round plate 422 of the attaching frame 42 is provided with an annular projection 429, similar to the round plate 122B of FIG. 7. Thereby, a gap through which liquid passes is formed between the round plate 422 of the attaching frame 42 and the plate substrate 411 of the fine needle plate 41.

In a state in which the fine needle plate 41 is attached to the attaching frame 42, the front end of the annular projection 429 contacts the back surface of the plate substrate 411, and a region enclosed by a front surface F25 of the round plate 422, the annular projection 429, and a back surface F24 of the plate substrate 411 becomes a partitioned gap space SF. Liquid enters the gap space SF from a flow path 425 of the attaching frame 42, resulting in forming an accumulation of liquid. Then, from the accumulation of liquid, the liquid spreads radially outward and flows into the discharge holes 416.

Subsequently, as illustrated in FIG. 17, the liquid entering the plurality of discharge holes 416 passes through the plate substrate 411 and the base 413, and is pushed out of the front recessed surface F13. The pushed-out liquid spreads in a space formed between the target object, the individual lip 415, and the front recessed surface F13 of the base 413, and enters the interior of the target object through the holes formed by the needles 414. Therefore, active ingredients of the liquid can penetrate into the target object O from both of the exterior and the interior of the target object O.

For example, the liquid having a beneficial effect on the target object is spread over the skin in the front recess of the base 413 in a state in which the skin is deformed and stretched by the front surface F15 of the individual lip 415. At the same time, the liquid penetrates into the target object through the holes formed by the needles 414 that are solid needles. Therefore, active ingredients of the liquid (e.g., beauty ingredients) can penetrate into the target object from both of the exterior of the surface of the target object and the interior of the target object.

At this time, as illustrated in FIG. 17, the gap enclosed by the target object and the front surface F15 of the individual lip 415 is small, and thus the liquid reaches the entire front recess of the base 413, i.e., a region enclosed by the target object and the front surface F15 of the individual lip 415. Specifically, in the case of orientation illustrated in FIG. 17, in the space enclosed by the skin and the front recess of the base 413, the liquid may first be accumulated in a lower part of the space, but eventually spreads upward as the space is filled with the liquid. In this manner, by preventing the liquid from leaking from the gap by the front surface F15 of the individual lip 415, the active ingredients can be retained on the surface of the target object and in the interior of the target object. Thereby, even if the syringe 80 is oriented upward or sideways, the active ingredients of the liquid can be provided on and in the surface of the target object by the fine needle part 4.

Here, in the present embodiment, height a15 from the plate substrate 411 to the front surface F15, i.e., a height from the plate substrate 411 to the vicinity of the outer periphery of the base 413, is 200 µm or more and less than 3,000 µm, and a height of projection (height difference) b14 of the needle 414 from the front surface F15 is preferably from 10 µm through 200 µm.

As illustrated in FIGS. 16 and 17, the plurality of lip-equipped two-step needles 412 are provided apart from each other. A pitch c14 between the apexes of the needles 414 is preferably 1 mm ≤ c14 < 5 mm.

In the present configuration, the outer diameter of the front surface F15 of the individual lip 415 formed integrally with the base 413 is preferably larger than 200 µm and 600 µm or less.

Considering deformation of the skin, a value ((a15+b14)/c14), i.e., a ratio of the total of the outer peripheral height (a15) of the base 413 and the height difference (b14) of the needle 414 to the pitch (c14) of the needles 414, is preferably 0.3 or more and 1.1 or less. In this case, preferably, the interval between the peripheries of the front surfaces F15 of the individual lips 415, i.e., the interval between the outer peripheries of the bases 413, is preferably 0.5 mm or more and 4.4 mm or less.

Also, the number of the lip-equipped two-step needles 412 per unit area on the plate substrate 411 is 0.1/cm² or more and 33/cm² or less.

### (Integrated configuration of the third embodiment)

FIG. 16 illustrates an example in which the fine needle plate 41 provided with the lip-equipped two-step needles 412 is a member separate from the attaching frame 42 provided with an attaching barrel 426 and the flow path 425, i.e., the fine needle part 4 is an assembled body. However, the fine needle part including the lip-equipped two-step needles 412 of the present embodiment may be a single portion in which a puncturing function is integrated with a function of attaching to the front barrel portion of the syringe, as in the fine needle part 1A of FIG. 6. Thus, according to the integrated fine needle part of the present embodiment, a plate support 421 is not provided, and a flow path is formed at the radial center of the fine needle plate 41, and an attaching barrel is provided integrally on the back side.

Further, in the present embodiment, the needle projects from the front end of the base. Thus, the shape of the needles of all or some of the lip-equipped two-step needles 412 may be changed.

### <Modified Example 1 of the needle of the third embodiment>

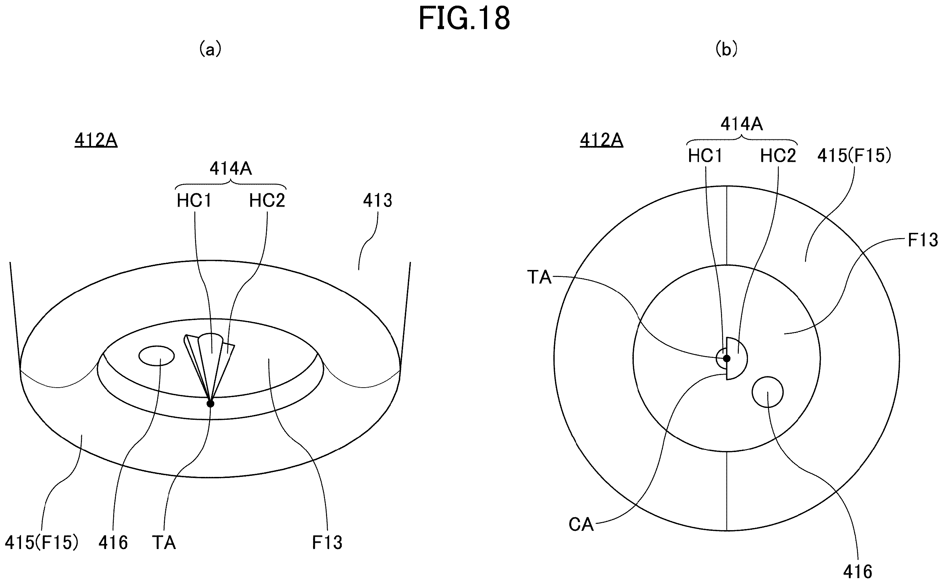

FIG. 18 is an explanatory view of Modified Example 1 of the needle in the lip-equipped two-step needle according to the third embodiment. In a lip-equipped two-step needle 412A of FIG. 18, (a) is a perspective view thereof and (b) is a bottom view thereof.

In the present configuration, as illustrated in FIG. 18, a needle 414A formed on the front recessed surface F13 of the base 413 in the lip-equipped two-step needle 412A has a shape in which two half cones (half-cut cones) HC1 and HC2 having different diameters face each other such that apexes TA coincide with each other.

With the present configuration, the cut surfaces of the half cones HC1 and HC2 face each other, and a part of the cut surface CA of the half cone HC2 having a larger diameter is exposed. Thus, the needle 414A has a surface area larger than that of the needle 414 of FIG. 16 that has a simple conical shape. As a result, the surface area of the side surface of the puncture hole of the target object punctured by this needle 414A increases, and the content is more likely to enter the interior of the skin. Thus, it is possible to promote penetration of the liquid from a single hole of the target object formed by the puncture of the needle. Therefore, in the case of performing a puncture with this needle 414A, it is possible to reduce the time required for liquid penetration at the time of the puncture, and to increase the extent of penetration into the target object compared to the configuration illustrated in FIG. 16.

In the present configuration example, the diameter of the smaller half cone is preferably from about 20% through about 70% of that of the larger half cone. For example, the radius of the bottom surface of the smaller half cone is preferably from 30 µm through 100 µm.

### <Modified Example 2 of the needle of the third embodiment>

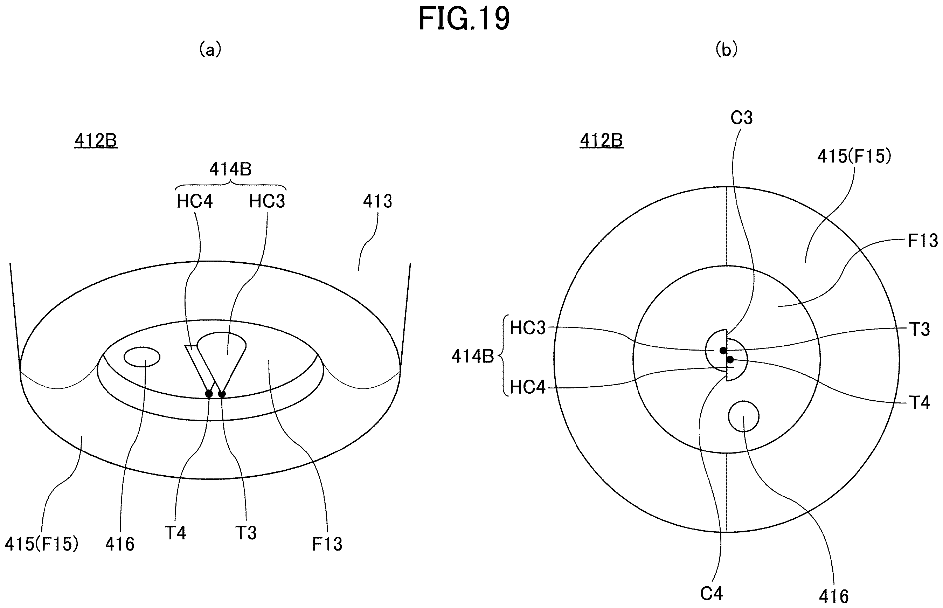

FIG. 19 is an explanatory view of Modified Example 2 of the needle in the lip-equipped two-step needle according to the third embodiment. In a lip-equipped two-step needle 412B of FIG. 19, (a) is a perspective view thereof and (b) is a bottom view thereof.

In the present configuration, as illustrated in FIG. 19, a needle 414B formed on the front recessed surface F13 of the base 413 in the lip-equipped two-step needle 412B has a shape in which halves of right cones (half-right cones) HC3 and HC4 are shifted in the direction along the cross-sectional surfaces, and face each other. In the present configuration, the center axes of the two facing half right cones HC3 and HC4 are shifted in the direction along the cross-sectional surfaces, and thus the positions of apexes T3 and T4 of the semi-right cones do not coincide with each other, and are apart from each other.

Therefore, in the present configuration example, there are two apexes T3 and T4 of the needle 414B. When the target object is punctured by the single lip-equipped two-step needle 412B, two holes are formed at close positions in the target object. This increases the number of sites for entry of fluid. Further, in the present configuration, parts of cut surfaces C3 and C4 are exposed at the facing surfaces of the half right cones HC3 and HC4. Thus, the needle 414B has a surface area larger than that of the needle 414 illustrated in FIG. 16 that has a simple conical shape. As a result, the content is more likely to enter the interior of the skin.

In the present configuration example, the diameters of the two facing half right cones HC3 and HC4 may be the same or different. When they are different, preferably, the diameter of the smaller half right cone is at least from about 60% through about 99% so as to come out from only one of the radial directions in the cross-sectional surface of the larger half cone. For example, the radius of the bottom surface of the half cone that is smaller or equal in size is preferably from 50 um through 100 µm.

### <Modified Example 3 of the needle of the third embodiment>

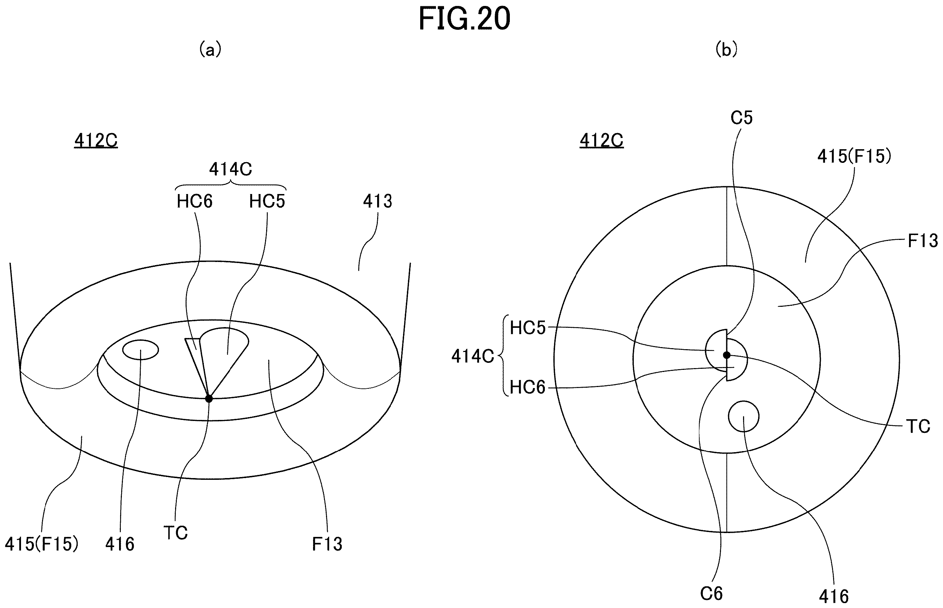

FIG. 20 is an explanatory view of Modified Example 3 of the needle in the lip-equipped two-step needle according to the third embodiment. In a lip-equipped two-step needle 412B of FIG. 20, (a) is a perspective view thereof and (b) is a bottom view thereof.

In the lip-equipped two-step needle 412C of the present example, as illustrated in FIG. 20, a needle 414C projecting from the front recessed surface 13F of the base 413 is formed of two oblique half cones HC5 and HC6 that are shifted in the direction of cross-sectional surfaces and face each other such that the positions of the apexes TC coincide with each other. The two oblique half cones HC5 and HC6 are each such that a line drawn to the apex from the center of the bottom surface of the cone is not perpendicular to the bottom surface.

In the present configuration, the cut surfaces of the oblique half cones HC5 and HC6 face each other such that parts of cut surfaces C5 and C6 of the oblique half cones HC5 and HC6 are exposed. Thus, the needle 414A has a surface area larger than that of the needle 414 of FIG. 16 that has a simple cone shape. As a result, the content is more likely to enter the interior of the skin. Also, the positions of the apexes are identical. Thus, the surface area of the interior of a single hole in the target object punctured increases, and the number of holes is not increased.

In the present configuration example, the diameters of the two facing oblique half cones HC5 and HC6 may be the same or different. When they are different, preferably, the diameter of the smaller oblique half cone is at least from about 60% through about 99% so as to come out from only one of the radial directions in the cross-sectional surface of the larger oblique half cone. For example, the radius of the bottom surface of the oblique half cone that is smaller or equal in size is preferably from 50 µm through 100 µm.

### <Fine needle plate of the fourth embodiment>

The above-described first to third embodiments describe the examples in which the needle of the two-step needle is a solid needle. However, the two-step needle in the fine needle part of the present invention may be a hollow needle.

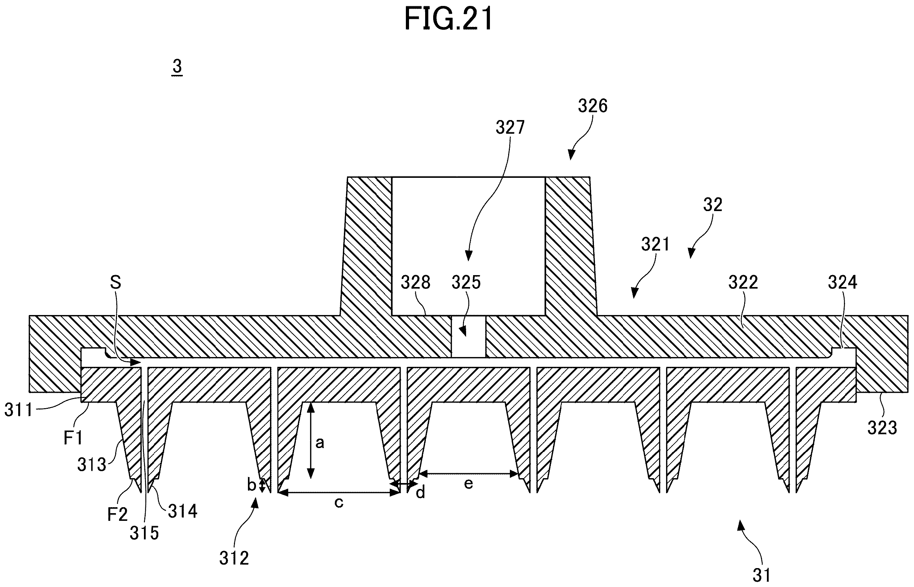

FIG. 21 is an enlarged cross-sectional view of a fine needle plate and an attaching frame according to the fourth embodiment. The fine needle part of the present embodiment includes: an attaching frame 32 including a flow path plate 322; and a fine needle plate 31.

As illustrated in FIG. 21, the fine needle plate 31 of the present embodiment includes a plate substrate 311, and a plurality of two-step needles 312 projecting from the front end of the plate substrate 311. The two-step needles include a plurality of bases (base portions) 313 and a plurality of needles 314. In the present embodiment, the plate substrate 311, which is a plate base (body), the base 313, and the needle 314 are integrally formed. The plurality of needles 314 project from the front surfaces of the plurality of bases 313. Also, a discharge hole 315 (see FIG. 21), which extends in the projection direction of the needle 314 and through which fluid passes, is formed so as to vertically penetrate through the needle 314, the base 313, and the plate substrate 311. That is, the two-step needle 312 of the present embodiment is a two-step hollow needle in which a through-hole penetrates through the base 313 and the needle 314.

The configuration of the attaching frame 32 is similar to that of the attaching frame 12 of the first embodiment.

Also, in the present embodiment, the fine needle plate 31 is pressed against the target object, such as skin or the like, in use. As illustrated in FIG. 1, in response to pushing of a back-end pusher 86 of the syringe 80, the pusher 85 is moved in the outer barrel 82 of the syringe barrel 81, and the content L is discharged from the syringe 80 into the fine needle plate 31 through the attaching frame 32.

In the present embodiment, when the content L reaches the front end of the front barrel portion 84 of the syringe 80, the content L is delivered to the target object, such as skin or the like, against which the fine needle plate 31 is pressed, after passage through a flow path hole 325 of the attaching frame 32 and then through the discharge hole 315 of the fine needle plate 31.

As illustrated in FIG. 21, the height (a) of the base 313 in the fine needle plate 31 is preferably 200 µm ≤ a < 3,000 µm. Also, the height (b) of the needle 314 is preferably 5 µm ≤ b < 600 µm.

In such a case, the total height (a+b) of the height (b) of the needle 314 and the height (a) of the base 313 is preferably from 600 um through 3,200 µm. Also, a value (b/a) of the ratio of the height (a) of the base 313 to the height (b) of the needle 314 is preferably 0.5 or less. Thus, the height (a) of the base 313 and the height (b) of the needle 314 are set, within the respective recommended ranges thereof, to such dimensions as to satisfy the above total value and the ratio.

As illustrated in FIG. 21, a front-end diameter (d) of the base 313 is preferably 300 µm ≤ d ≤ 600 µm, and the diameter at the root of the needle 314 is set to be smaller than the front-end diameter of the base 313. Also, in order to improve skin contact, the edge of the front surface (top surface) F2 of the base 313 is preferably rounded.

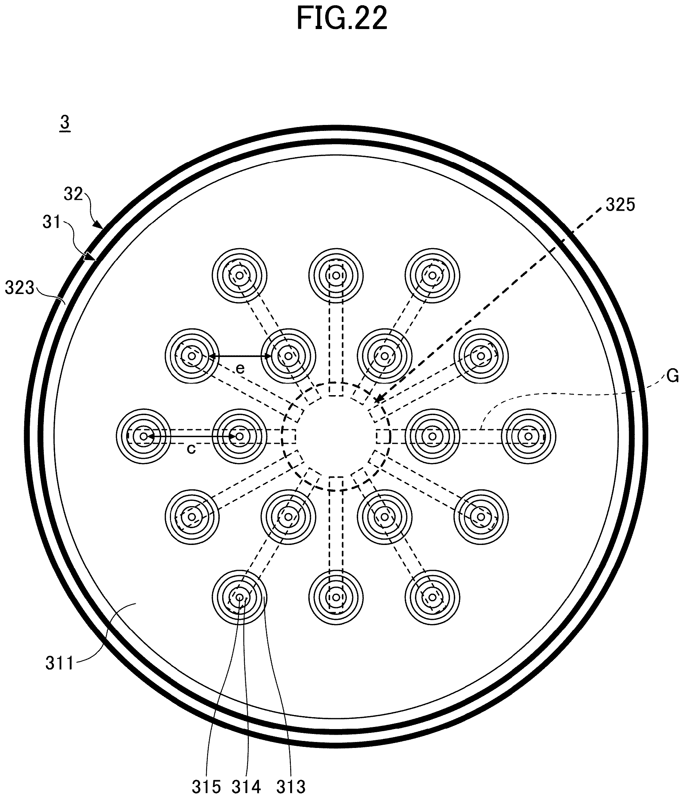

FIG. 22 is a bottom perspective view of an example of the fine needle part 3 of the present invention. According to the fine needle plate 31 of the present invention, the number of the two-step needles 312, each being a base-equipped needle in which the base 313 and the needle 314 are formed integrally, may be any number, and is more preferably two or more. When a plurality of two-step needles 312 are provided, the two-step needles are preferably arranged orderly. FIG. 22 illustrates an example in which the apexes of the needles 314 are arranged in an oblique lattice (triangular arrangement) in a bottom view of the fine needle plate 31. However, the apexes of the needles 314 may be arranged in a lattice (rectangular arrangement).

As illustrated in FIGS. 21 and 22, when the plurality of two-step needles 312, each being a set of the base and the needle, are provided so as to project from the plate substrate 311, preferably, the bases 313 are disposed apart from each other, and a pitch (c) of the apexes of the needles 314 is 1 mm ≤ c < 5 mm.

The value ((a+b)/c), i.e., the ratio of the total of the height (a) of the base 313 and the height (b) of the needle 314 to the pitch (c) of the needles 314, is preferably 0.3 or more and 1.1 or less. In this case, the interval (e) between the edges of the front surfaces F2 of the bases 313 is 0.5 mm or more and 4.4 mm or less.

Also, the number of the bases 313 per unit area on the plate substrate 311 is preferably 0.1/cm² or more and 33/cm² or less.

According to the fine needle part 3 according to the present configuration example, the liquid flowing from an attaching barrel 326 passes through the flow path hole 325 at the center of a perforated bottom surface 328, and spreads as an accumulation of liquid in a space S on the upper surface of the plate substrate 311 and then enters the plurality of discharge holes 315.

The liquid entering the plurality of discharge holes 315 penetrates through the interior of the target object through the fine needles 314. At this time, in the present embodiment, the bases 313 are provided integrally with the needles 314, and thus push and stretch the target object, such as skin. This is expected to improve puncture performance.

In the present embodiment, as denoted with the dotted line in FIG. 22, flow paths may be radially provided at the upper surface of the plate substrate 311. Details of the flow path will be described below with reference to FIG. 23.

The fine needle plate 31 including the two-step needles 312 of the present invention is preferably formed of a biocompatible material including a biodegradable resin, such as polyglycolic acid, polylactic acid, a polyglycolic acid-polylactic acid copolymer, or the like. However, the fine needle plate 31 may also be formed of a thermoplastic resin, such as polypropylene (PP), polyethylene (PE), acrylonitrile butadiene styrene (ABS), polybutylene terephthalate (PBT), polyacetal (POM), polyethylene terephthalate (PET), polycarbonate (PC), polyether ether ketone (PEEK), cycloolefin polymer (COP), or the like. In addition, biocompatible metal materials, such as stainless steel, cobalt alloys, titanium alloys, and the like, silicone, ceramics, and the like may also be used.

The attaching frame 32 according to the present embodiment is preferably formed of a resin, such as, for example: a thermoplastic resin, e.g., PP, PE, ABS, PBS, POM, PET, PC, PEEC, COP, or the like; or a biodegradable resin, e.g., polylactic acid (PLA), polyglycolic acid (PGA), a polylactic acid-glycolic acid copolymer (PLGA), or the like.

### <Second configuration example of the fine needle part of the fourth embodiment>

In the above example, the upper surface of the plate substrate 311 and the lower surface of the flow path plate 322 are both flat surfaces. However, in order to more efficiently move the liquid radially outward from the center, a communication path may be formed.

A configuration in which a communication path is formed in the integrally hollow fine needle part 3 of the present embodiment will be described with reference to FIGS. 22 and 23. FIG. 23 is an enlarged cross-sectional view of a fine needle part 3A of the second configuration example of the fourth embodiment.

In the present configuration example, as illustrated in FIG. 22, a fine needle plate 31A includes the plurality of two-step needles 312, each being a base-equipped needle that is a set of the base 313 and the needle 314, so as to project from a plate substrate 311A. The two-step needles 312 are provided at positions other than the center of the plate substrate 311A.

In FIG. 22, which is a bottom view, dotted lines radially extending from the flow path hole 325 are communication paths G. In the present configuration example, as illustrated in FIGS. 22 and 23, the communication paths G are formed by forming grooves (band-shaped recesses) at an upper surface (second surface) F0 of the plate substrate 311 in the region of the shortest path through which liquid flows from the center to the plurality of discharge holes 315. At this time, as illustrated in FIG. 23, the configuration may be such that the upper surface F0 external of a groove 316 of the plate substrate 311 is contacted with a lower surface F3 of the flow path plate 322 and no gap except for the groove 316 is formed.

As a further modified example of the present example, the upper surface F0 of the plate substrate 311A is contacted with the lower surface F3 of the flow path plate 322, thereby radially forming a band-shaped groove (not illustrated) at the lower surface F3 of the flow path plate 322 as illustrated in FIG. 22, and forming the communication paths G as a space through which liquid passes.

In this second configuration example, the communication paths are formed by providing the radial grooves in no contact with the surface of the plate substrate 311A or the flow path plate 322. Thus, the plate substrate 311A excluding the grooves can be contacted with the flow path plate 322, as illustrated in FIG. 23. Therefore, the dead space in the fine needle part can be reduced, and the size can be reduced.

### <Third configuration example of the fourth embodiment of the fine needle part>

FIG. 23 illustrates an example in which the communication paths are formed by providing radial grooves at the plate substrate 311 or the flow path plate 322. However, the communication paths may be formed by providing other members between the plate substrate 311 and the flow path plate 322.

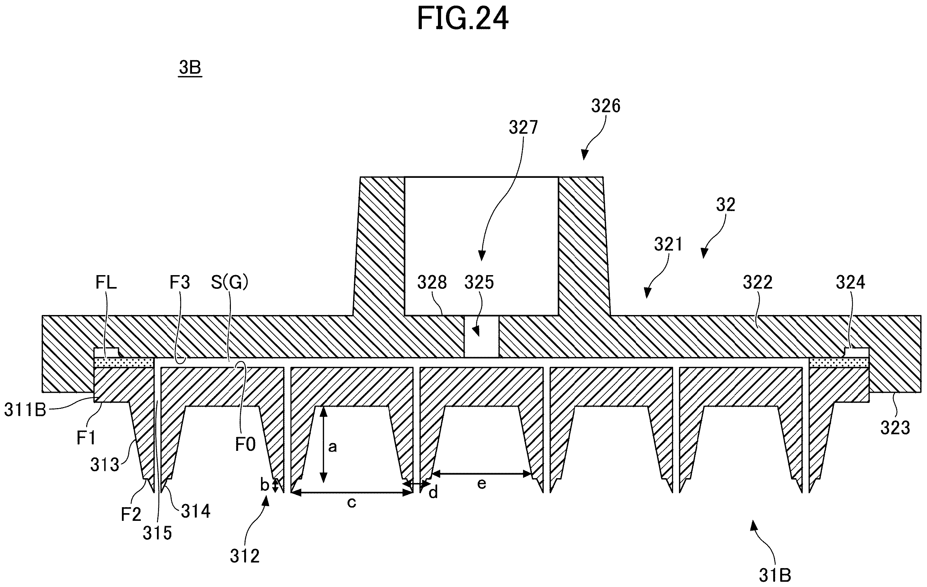

FIG. 24 is an enlarged cross-sectional view of the fine needle part 3B of the third configuration example of the present embodiment. In the present configuration example, the communication paths G are formed, as a space through which liquid passes, by providing a gap space S with a filler FL in a portion other than the region of the shortest path through which liquid flows from the center to the plurality of discharge holes 315. The gap space S is formed by disposing the upper surface F0 of the plate substrate 311B in proximity to the entire area of the lower surface F3 of the flow path plate 322.

As illustrated in FIGS. 22 and 24, by providing the communication paths G without providing the needle at the center, the liquid can be more efficiently moved radially outward in a straight line from the center without spreading the liquid over the entire area of the gap space. Thus, the liquid can be more quickly and substantially uniformly delivered to the target object from all of the needles. Further, by providing the communication paths G, the liquid does not stay in the entire gap space, thereby reducing waste of the liquid and delivering the liquid content to the target object as much as possible. Also, the space to be filled with the liquid is small, and thus the action of injection for filling the space at the initial action of injection is not needed so much, and the liquid can be immediately injected into the skin.

Alternatively, as a method for completely uniformly discharging the liquid from all of the needles, the communication paths may be designed such that the length from the center flow path hole 25 to each of the needle holes is the same. For example, the communication path may be formed so as to detour when it is close to the center, and the communication path may be formed in a straight line when it is close to the outer periphery.

### <Application Example 2: Knock-type injection device>

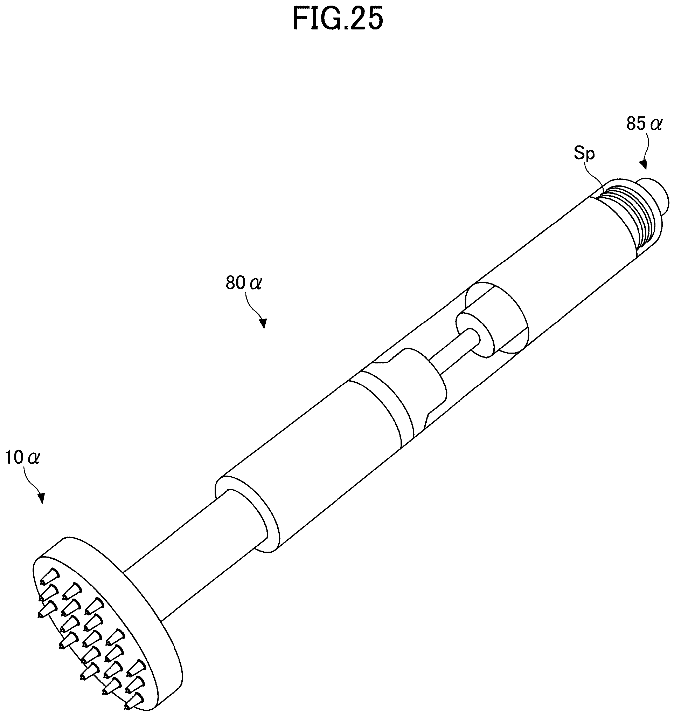

FIG. 25 is an overall view of a knock-type injection device that is Application Example 2 in which the fine needle part according to the embodiment of the present invention is attached.

A knock-type injection device 80α illustrated in FIG. 25 has the same connection configuration with the fine needle part on the front side as that illustrated in FIG. 1, except that by knocking a press portion 85α on the back side, an internal spring Sp pushes the content by a predetermined length and a predetermined amount thereof is pushed out.

FIG. 1 illustrates a syringe as an example of a skin puncture instrument to which the fine needle part having any configuration of the present invention is attached. In the case of using a syringe, for applying an appropriate amount in a divided manner, i.e., twice or more, it is necessary to push the pusher for discharging the liquid while reading the scale. However, the present application example can apply a fixed amount in a divided manner, i.e., twice or more, by use of the knock type.

FIG. 25 illustrates an example of an injection device configured to apply a fixed amount by use of the knock type. However, the fine needle part to which the fine needle part of the present invention is to be applied may be, for example, a dial-type injection device configured to apply a fixed amount by rotating a shaft in the circumferential direction. A knock portion or a dial portion may be provided on the side surface of the shaft.

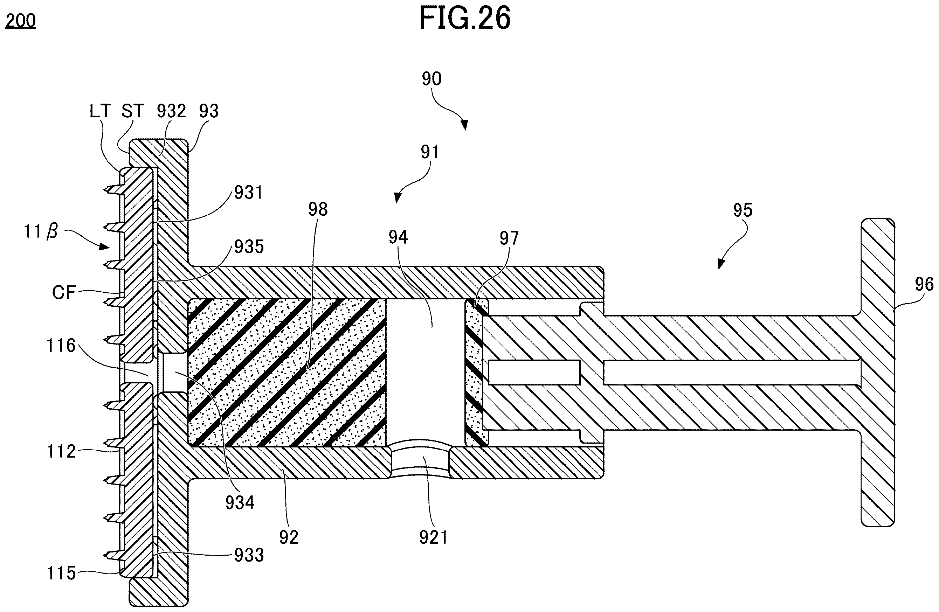

The present application example uses the same attaching method as that in FIG. 1. Thus, any of the fine needle parts of the first, second, third, and fourth embodiments described above can be attached to the knock-type injection device 80α of FIG. 26.

### <Application Example 3: Injection device>

Next, an injection device to which the fine needle plate of the present invention is attached will be described with reference to FIG. 26. The fine needle plate 11 of the present invention can be attached to an injection device 200 in use. FIG. 26 is an overall cross-sectional view of the injection device 200, in which a fine needle plate 11β according to the embodiment of the present invention is attached to an injection mechanism 90.

According to the present application example, the injection mechanism 90, to which the fine needle plate 11β is attached, includes an injection device body 91 and a pusher 95. The injection device body (injection guide) 91 in the present configuration houses a liquid content, and the pusher 95 is inserted into the body. Specifically, the injection device body 91 includes: a cylindrical outer barrel 92 configured to house the content; and a head (pressing portion, supporting portion) 93 that extends in a brim shape toward the outer periphery on the front side.

As illustrated in FIG. 26, the front surface of a head 93 includes: a thin plate 931 to serve as the top surface for housing; and an outer peripheral thick portion 932 to serve as a side wall for housing. With this configuration, a recess is formed on the front side, and a step is formed between the thin plate 931 and the outer peripheral thick portion 932. The inner surface of the outer peripheral thick portion 932 forms a recessed side surface 933. A communication hole 934 is formed at the center of the thin plate 931 of the head 93. According to this application example, the thin plate 931 serves as a flow path plate.

According to this configuration, in attaching the fine needle plate 11β to the injection mechanism 90, the outer periphery of the fine needle plate 11β is fitted radially inward of the recessed side surface 933 on the lower surface side of the head 93.

Also, the outer peripheral surface of the outer barrel 92 may be provided with a side-surface hole 921, as illustrated in FIG. 26. For example, when the same person increases the quantity of the content to be applied to the target object per use, the content is injected into the outer barrel 92 through the side-surface hole 921 using, for example, a dropper. At this time, the quantity of the content is set to be below the side-surface hole 921.

The back end of the pusher 95 (plunger) is provided with a pusher 96 that has a widened brim shape or a wing shape extending in two directions. Also, a gasket 97 adheres to the front end of the pusher 95, where the gasket 97 is a thin-plate packing formed of rubber or elastomer for preventing liquid leakage and contamination. In the present configuration, the pusher 95, the pusher 96, and the gasket 97 are pushers. The gasket 97 may or may not be provided.

Moreover, in the present application example, the inner periphery of the outer barrel 92 defines a hollow portion 94. Although FIG. 26 illustrates a state in which an absorbent 98 such as a sponge is set in the hollow portion 94 of the outer barrel 92, the absorbent 98 may or may not be provided in the present application example.

With this configuration, by pushing the pusher 96 of the pusher 95 in a state in which the fine needle plate 11 is pushed onto the target object, the pusher 95 descends inside the outer barrel 92, and the liquid that has been previously injected to the hollow portion 94 through the side-surface hole 921 passes through the absorbent 98. Subsequently, the liquid is discharged through the communication hole 934 and the discharge hole 14, and gradually applied to the target object. Note that, when the absorbent 98 is not provided, the liquid previously injected into the hollow portion 94 is immediately discharged through the communication hole 934 and the discharge hole 14 of the fine needle plate 11, and applied to the target object.

According to the present application example, the portion corresponding to the attaching frame 12 is integrated with the barrel of the injection device. Thus, in terms of shape, the fine needle part 1 or 3 of the above-described first, third, or fourth embodiment is preferable.

### <Application Example 4: Needle applicator>

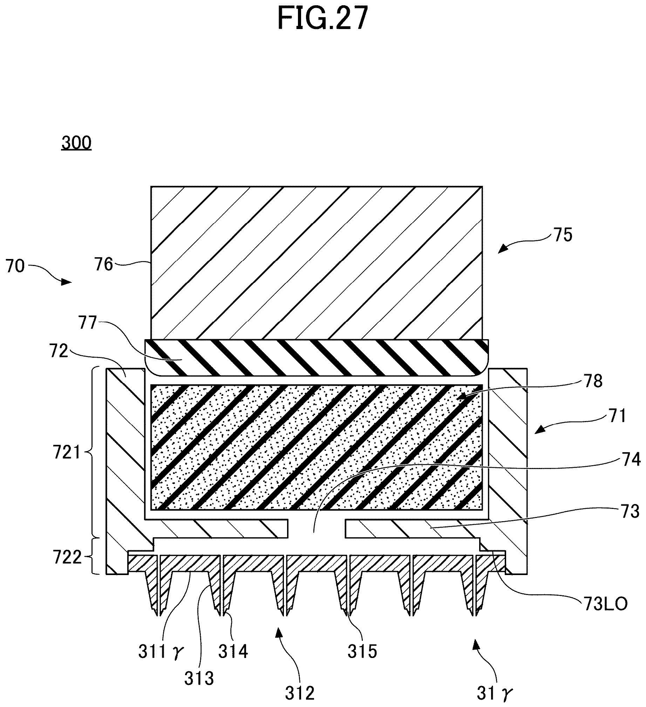

Next, a needle applicator to which the fine needle plate of the present invention is attached will be described with reference to FIG. 27. FIG. 27 is an overall cross-sectional view of a needle applicator 300 to which a fine needle plate 31γ according to the fourth embodiment of the present invention is attached.

The needle applicator 300, to which the fine needle part of the present invention is to be attached, is a stamp-type puncture injector configured to deliver liquid into the target object (e.g., skin) through the interior of a plurality of fine needles.

As illustrated in FIG. 27, the needle applicator 300 includes a gripping casing 71, the fine needle plate 31γ, an absorbing member 78, and a pressing member 75. The portion of the needle applicator 300 excluding the fine needle plate 31γ is an applicator portion 70.

The gripping casing 71 is a support (housing) having an upper opening, and includes a side wall 72 and a supporting wall 73. The side wall 72 is a cylindrical peripheral wall. The supporting wall 73 is a lower surface that is continuous with the inner surface near the lower end of the side wall 72, extends in a direction approximately orthogonal to the side wall 72, and covers the lower portion of the gripping casing 71.

With the supporting wall 73 serving as a boundary, the side wall 72 is divided into: a housing wall 721 on the upper side (proximal side) of the supporting wall 73; and a leg portion 722 on the lower side (distal side) of the supporting wall 73. The lower portion of the leg portion 722 of the side wall 72 is thinner in the inner portion than the upper portion thereof, thereby forming a step. As a result, the lower periphery of the supporting wall 73 is a thick lower surface, and that portion serves as a liquid stopper wall 73LO.

In the present application example, a single vertically penetrating flow hole 74 is formed at the center of the supporting wall 73. In this configuration, the supporting wall 73 serves as a flow path plate.

The gripping casing 71 is formed of a resin, such as, for example, a thermoplastic resin, e.g., PP, PE, ABS, PBT, POM, PET, PC, PEEK, COP, or the like, or a biodegradable resin, such as polylactic acid (PLA), polyglycolic acid (PGA), a polylactic acid-glycolic acid copolymer (PLGA), or the like. In addition, biocompatible metal materials, such as stainless steel, cobalt alloys, titanium alloys, and the like, silicone, ceramics, glass, and the like may also be used.

The needle applicator 300 of the present application example is small. For example, the outer diameter of the side wall 72 of the gripping casing 71 is from about 8 mm through about 50 mm, and more preferably from about 10 mm through about 40 mm. Although the outer shape of the side wall 72 is not illustrated in the present example, the outer shape of the side wall 72 in a top view may be any of different shapes, such as a circle, an ellipse, a rectangle, a polygon, a rounded polygon, or a substantial semi-circle.

The height of the side wall 72 of the gripping casing 71 is from about 6 mm through about 30 mm, and more preferably from about 8 mm through about 20 mm. When the side wall 72 is 6 mm or more, a user can grip the side wall 72 from radially outward. The thickness of the side wall 72 is from about 1 mm through about 10 mm, and more preferably from about 1 mm through about 5 mm.

The fine needle plate 31γ is a plate provided with a plurality of downward-facing fine needles by which the target object is to be punctured. In the present application example, the fine needle plate 31γ is fitted into the inner periphery of the thin wall portion of the leg portion 722 of the gripping casing 71. Thereby, as illustrated in FIG. 27, the fine needle plate 31γ is attached to the gripping casing 71 on the lower side of the lower surface of the supporting wall 73 with a gap of the liquid stopper wall 73LO.

The configuration and material of the fine needle plate 31γ of the present application example are the same as those in the above-described fourth embodiment. However, in the present configuration, in order for a plate substrate 311γ of the fine needle plate 31γ to be fitted into the side wall 72 of the gripping casing 71, the outer diameter of the plate substrate is from about 6 mm through about 48 mm, and more preferably from about 8 mm through about 36 mm.

The absorbing member 78 is impregnated with liquid. The absorbing member 78 may be impregnated with liquid in advance or immediately before use. The absorbing member 78 can be fitted radially inward of the side wall 72 of the gripping casing 71 and into the upper portion (back side) of the supporting wall 73. When fitting the absorbing member 78 into the gripping casing 71, the lower surface of the absorbing member 78 is preferably set to contact the upper surface of the supporting wall 73.

The absorbing member 78 is formed of a permeable material formed of a material through which liquid gradually passes, such as a sponge (puff), cotton, nonwoven fabric, or the like.

The pressing member 75 serves substantially as a pusher. The pressing member 75 includes a columnar pressing body 76, and an elastic portion 77 provided at the lower end of the pressing body 76. A part of the pressing member 75 can be fitted into the interior of the side wall 72 of the gripping casing 71. The pressing member 75 is fitted over the absorbing member 78 in the gripping casing 71, and pressed along the inner peripheral surface of the side wall 72.

When injecting liquid into the target object in use, a user presses the upper surface of the pressing body 76. The pressing body 76 is formed of a resin, such as, for example, a thermoplastic resin, e.g., PP, PE, ABS, PBT, POM, PET, PC, PEEK, COP, or the like, or a biodegradable resin, such as polylactic acid (PLA), polyglycolic acid (PGA), a polylactic acid-glycolic acid copolymer (PLGA), or the like. In addition, biocompatible metal materials, such as stainless steel, cobalt alloys, titanium alloys, and the like, silicone, ceramics, glass, and the like may also be used.

The elastic portion 77 is configured to seal the absorbing member 78 from above when the pressing member 75 is fitted onto the absorbing member 78. The elastic portion 77 is formed of, for example, a packing (hermetically sealing element) formed of rubber or elastomer in the form of a thin plate. The elastic portion 77 formed of such a material can prevent liquid leakage and contamination of foreign matter.

The diameter of the pressing member 75 is, for example, from about 10 mm through about 30 mm, and more preferably from about 15 mm through about 25 mm. The height of the pressing member 75 is 30 mm or less, and more preferably 25 mm or less. However, the size of the pressing member 75 is appropriately set in accordance with the size of the side wall 72 of the gripping casing 71. In particular, the elastic portion 77 is set to a size that is attachable to the inner peripheral surface of the side wall 72. Also, the shape of the upper surface of the pressing member 75 is appropriately set in accordance with the outer shape of the side wall 72 in a top view.

Also, the pressing member 75 may be formed to have a shape that is not to be pushed straight but rather is to be pushed by rotation. A part of the pressing member 75 (internal thread) is fitted to and screwed in the outer side surface of the gripping casing 71 (external thread), whereby the absorbing member 78 is pushed, followed by injection. In this case, the pressing member 75 may be formed to have a shape that is fitted thereto and screwed therein before injection, and in which injection can be performed by pressing a button at the time of injection.

In the present application example, one type of content contained in the absorbing member 78 in advance can be injected to the target object. Alternatively, in addition to the content contained in the absorbing member 78 in advance, an additionally injected liquid content is contained before setting of the pressing member 75, and may be injected into the target object while mixing two types of contents.

Alternatively, the needle applicator 300 in the present application example may be assembled immediately before use and additionally supplemented with a content, thereby forming a puncture injection kit configured to apply liquid containing two types of contents to the target object. In this case, the puncture injection kit is a single-use product.

In the present application example, the attaching frame is integrated with the barrel of the injection device. Thus, the fine needle part 1 or 3 of the above-described first, third, or fourth embodiment is preferable.

### <Application Example 5: Head push-in injection device>

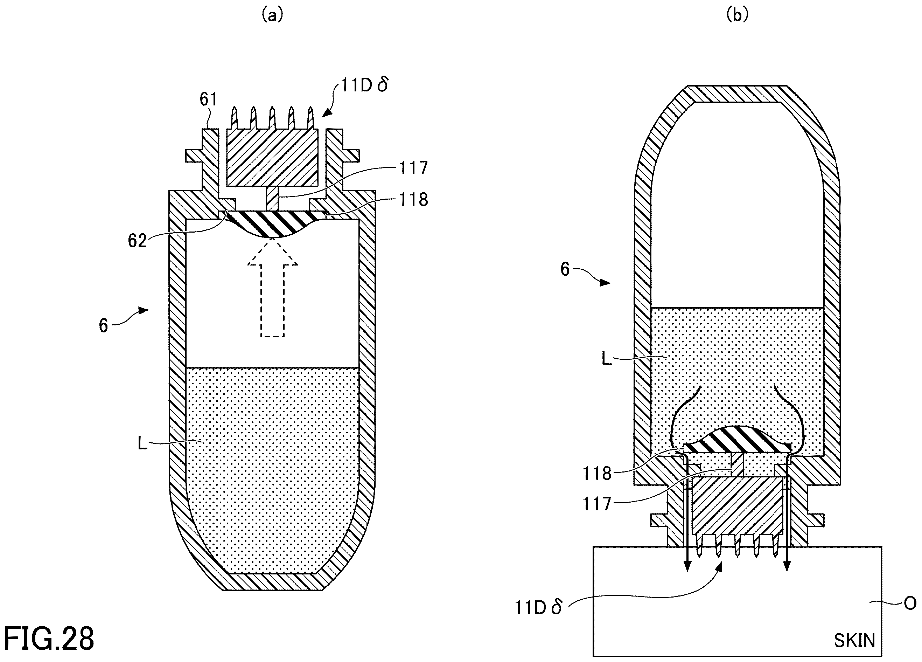

FIG. 28 is an overall cross-sectional view of a head push-in injection device that is Application Example 5 in which the fine needle part according to the embodiment of the present invention is attached.

The above application examples describe the examples in which the liquid is pushed out in response to pushing of the back end with the pusher or the knock portion. However, the present invention may be applied to a skin puncture instrument in which the back end thereof is not pushed with a pusher or the like.

According to the present application example, the attaching frame is integrated with the barrel of the injection device, and further, the liquid is applied to the skin in response to pushing of the front surface. Thus, the present application example uses a fine needle plate 11Dδ having the same functions as those of the fine needle plate 11D of the fifth configuration example of the first embodiment illustrated in FIGS. 9 and 10, i.e., the fine needle plate including the integrated solid needles. The fine needle plate 11Dδ of the present application example is preferably small. The outer dimensions are, for example, from about 8 mm through about 50 mm, and more preferably from about 10 mm through about 40 mm.

According to an injection device 6 illustrated in FIG. 28, the fine needle plate 11Dδ can be raised and lowered along the inner circumference of a cylindrical opening 61. Also, a plurality of projections 62, projecting toward the center and arranged in the circumferential direction, are provided inward of the inner circumferential surface of the cylindrical opening 61.

In the non-use state illustrated in (a) of FIG. 28, the sealing valve 118 pushed up by the action of a force of an internal spring or the like contacts the plurality of projections 62, thereby preventing the fine needle plate 11Dδ from projecting out of the cylindrical opening 61. Also, the sealing valve 118 closely contacts the inner wall of the cylindrical opening 61, thereby preventing leakage of the liquid content.

Meanwhile, upon use illustrated in (b) of FIG. 28, when the fine needle plate 11Dδ is pressed against the target object, such as skin, the fine needle plate 11Dδ is pushed into the cylindrical opening 61, and the sealing valve 118 is moved to release the close contact state between the sealing valve 118 and the inner wall of the cylindrical opening 61. Also, the projections 62 exist intermittently without covering the entirety in the circumferential direction. Thus, the liquid passing outward (radially outward) of the sealing valve 118 passes through the gap between the plurality of projections 62, and flows into the skin from the outer side of the outer periphery of the fine needle plate 11Dδ.

The needles formed at the fine needle plate 11Dδ are two-step needles. Thus, the skin, i.e., the puncture target, can be reliably punctured by the fine needles without being left to stand for a long time regardless of deformation of the skin. Then, the liquid flowing from the outer side toward the center in the radial direction can be applied to the skin.

### <Application Example 6: Puncture application roller>

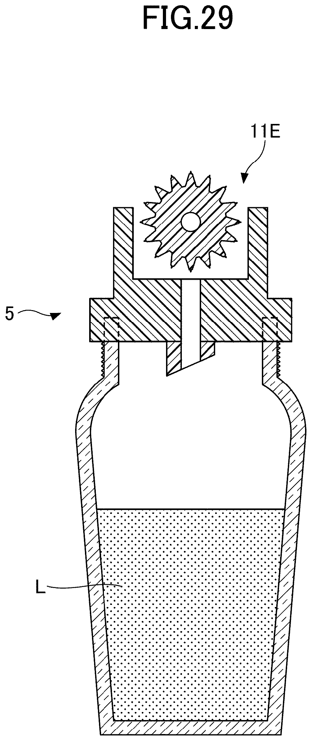

FIG. 29 is an overall explanatory view of a puncture application roller that is Application Example 6 in which the fine needle part according to the embodiment of the present invention is attached.

The above application examples and embodiments illustrate the fine needle part including the fine needle plate in which the plate substrate is a flat substrate. However, a fine needle plate 11E may be cylindrical.

The fine needle plate 11E of the present application example is preferably small. Regarding the outer dimensions thereof, the width of a barrel, i.e., the outer diameter of the barrel, is from about 8 mm through about 50 mm and more preferably from about 10 mm through about 40 mm, and the transverse length of the barrel is from about 8 mm through about 50 mm and more preferably from about 10 mm through about 40 mm.

According to the present application example, the needles formed at the fine needle plate 11E are two-step needles, and the plate is cylindrical. Thus, regardless of deformation of the skin, i.e., the puncture target, the roller is rolled without being left to stand for a long time, and the skin can be reliably punctured by the fine needles and the liquid can be applied to the skin.

FIG. 29 illustrates the application roller in which a puncture tool is integrated with a housing configured to house liquid. However, the puncture tool may be a member separate from the housing.

### <Application Example 7: Puncture injection set>

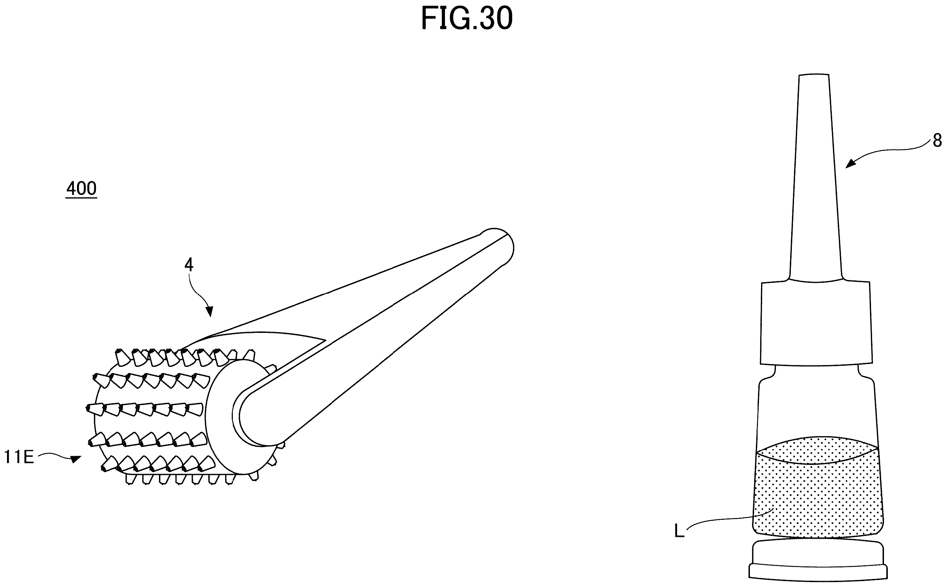

FIG. 30 is an overall explanatory view of a puncture injection set 400 including a separate liquid-type puncture application roller 7 and a housing container 8, i.e., Application Example 6 in which the fine needle plate according to the embodiment of the present invention is attached. According to the present application example, a beauty tool including the two-step needle-equipped fine needle plate 11E formed on the outer peripheral surface thereof includes a grasping tool 5 to be grasped by a user. The housing container 8 houses, for example, liquid containing beauty ingredients or the like.

When using the present puncture injection set, the target object is punctured by the needles after or before application of the liquid housed in the housing container to the target object, thereby applying the liquid onto the surface of the target object or to the interior of the target object.

In this case, the needles formed at the fine needle plate are two-step needles. Thus, regardless of deformation of the skin, i.e., the puncture target, the roller is rolled without being left to stand for a long time, and the skin can be reliably punctured by the fine needles and the liquid can be applied to the skin.

As described above, the fine needle part and the fine needle plate of the present invention can be attached to various puncture instruments, such as a syringe, an injection mechanism, and an applicator mechanism as illustrated in the above embodiments, in accordance with applications, desired puncture areas, and quantities of a content to be injected.

### <Experimental Examples>

The inventors of the present application performed experiments by using various dimensions in order to study the optimum value of the size of the needle in the fine needle plate. In the following experiments, the skin separated from a human body was used as the puncture object for evaluation of puncture performance. Specifically, a texture analyzer was used to push a fine needle plate in the skin stratum corneum at a load of 1,000 gf applied perpendicular to the surface of the skin horny layer using a texture analyzer. Subsequently, the number of needles that reached the epidermal granular layer or thereunder was measured through staining using an aqueous dye.

### <Experimental Example 1>

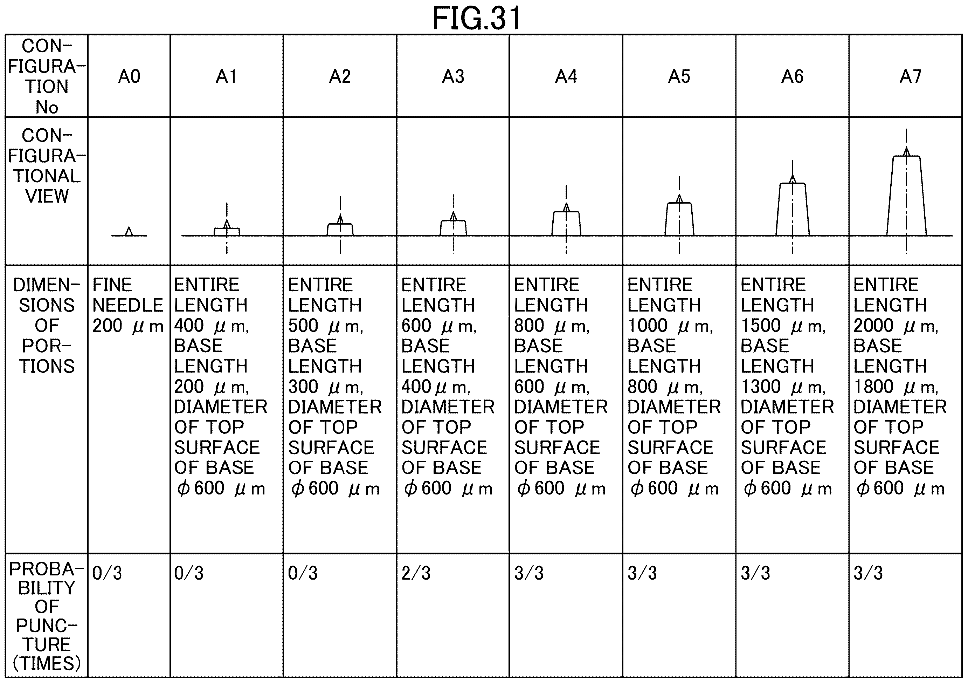

Puncture experiments were performed by varying the length of the base. FIG. 31 is a table illustrating results of the puncture experiments of the fine needle plate of the first configuration example of the first embodiment using the bases of different lengths. In Experimental Example 1, the puncture experiments were performed on the fine needle plate having a configuration in which the number of needles or base-equipped needles was one.

The thickness of the human skin is from about 1.0 mm through about 4.0 mm, and the thickness of the epidermis thereof is from about 0.02 mm through about 0.2 mm. In particular, there is a report stating that the thickness of the epidermis of the face is from about 0.02 mm through about 0.12 mm, and the thickness of the dermis is from about 0.3 mm through about 2.7 mm. In an attempt to apply a content into the epidermis and the dermis, the height of the needle 314 is preferably 5 µm ≤ b < 700 um. In this experiment, the length of the needle was fixed to 200 µm (0.2 mm) .

As illustrated in FIG. 31, the puncture performance of the fine needle plate is improved by providing the base. In particular, the puncture performance of configurations A3, A4, A5, A6, and A7 is especially good. Therefore, in the fine needle plate 11, the height (a) of the base 113 is preferably 200 µm ≤ a < 3,000 pm, and more preferably 400 µm ≤ a < 2,000 µm.

### <Experimental Example 2>

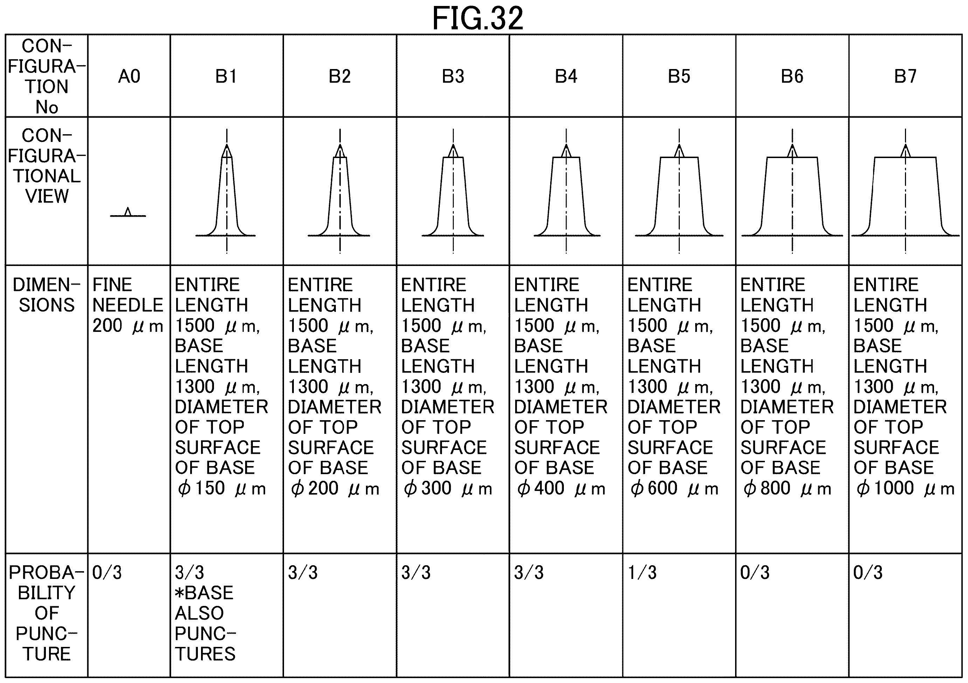

Puncture experiments were performed by varying the diameter of the base. FIG. 32 is a table illustrating results of the puncture experiments of the fine needle plate of the first configuration example using the bases of different front-end diameters. In Experimental Example 2, the puncture experiments were performed on the fine needle plate having a configuration in which the number of needles or base-equipped needles was only one.

As illustrated in FIG. 32, the smaller base 113 is more preferable. In particular, configurations B1, B2, B3, B4, and B5 exhibit good puncture performance. However, the base 113 of B1 is formed continuously with the needle 114 at the tip thereof, and thus the skin was also punctured by the base 113. Therefore, the front-end diameter (d) of the base 113 is preferably 200 µm < d ≤ 600 um, and the diameter at the root of the needle 114 is set to be smaller than the front-end diameter of the base 113.

### <Experimental Example 3>

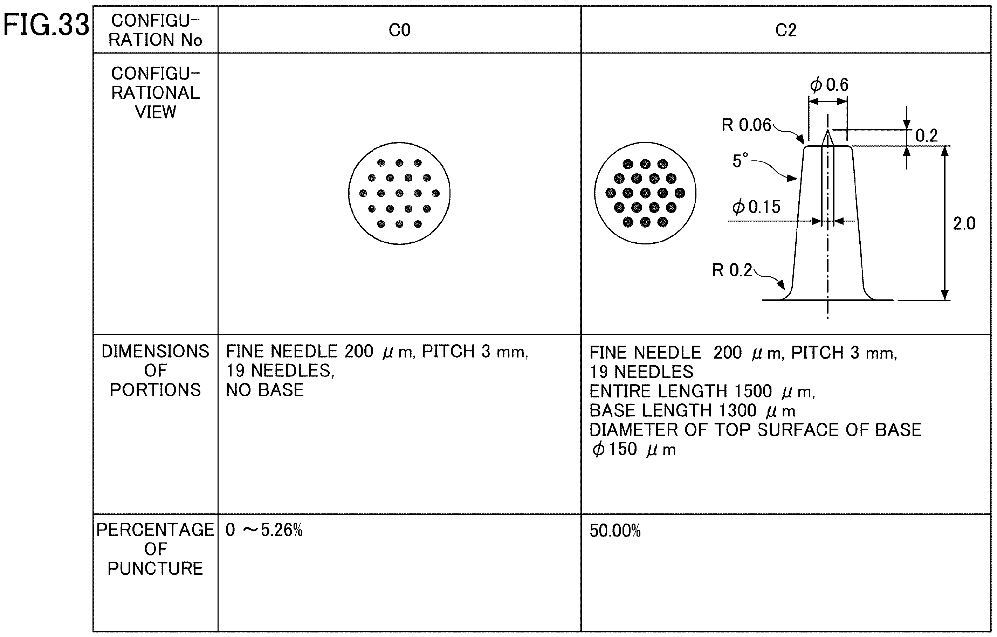

Puncture experiments were performed on configurations in which a plurality of needles were provided and bases were or were not provided. FIG. 33 is a table illustrating results of the puncture experiments of the fine needle plate with and without the bases, in which the plurality of needles were arranged in rows (in an array).

Even if the plurality of needles are arranged orderly, the skin is not readily punctured by the needles without the bases, while the skin is readily punctured by the needles with the bases. This indicates that the presence of the bases 113 increases the percentage of puncture. Specifically, the bases 113 push and stretch the surface of the elastic target object, such as skin, and the stretched skin is punctured by the needle 114. Thus, even if the plurality of needles are arranged in an array, the front surfaces F2 of the bases 113 contact the target object and stretch the surface of the target object.

### <Experimental Example 4>

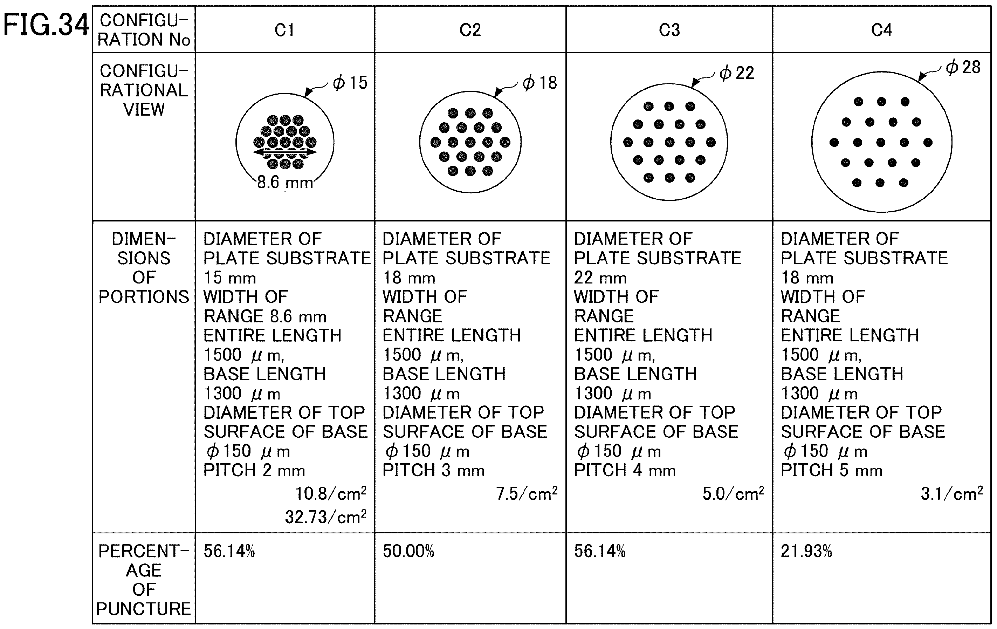

Puncture experiments were performed by providing a plurality of needles and varying the pitch thereof. FIG. 34 is a table illustrating results of the puncture experiments when the plurality of base-equipped needles are arranged in rows, the number of needles is the same, and the pitch thereof is varied.

As illustrated in FIG. 34, when the pitch is 2 mm, 3 mm, and 4 mm, the percentage of puncture is good. However, when the pitch is 5 mm, the percentage of puncture decreases. The bases 113 push and stretch the surface of the elastic target object. However, when the bases 113 are disposed at broad intervals, conceivably, the target object tends to gather at regions including no base-equipped needles, i.e., regions between the peripheries of the front surfaces F2 of the bases 113, and as a result, the effect of pushing and stretching the target object is weakened. Therefore, the pitch (c) of the apexes of the needles 114 is preferably 1 mm ≤ c < 5 mm, and more preferably 2 mm ≤ c < 4 mm. However, when the pitch is broad but the front-end diameter of the bases 113 is large, the intervals between the peripheries of the front surfaces F2 of the bases 113 are not so large. Therefore, the interval (e) between the edges of the front surfaces F2 of the bases 113 is preferably 0.5 mm or more and 4.4 mm or less.

Also, the depth by which the target object is pushed in pushing and stretching the target object varies in accordance with the height (a) of the base 113. Thus, considering the total length of the height (a) of the base 113 and the height (b) of the needle 114, the value ((a+b)/c) of the ratio of the total of the height (a) of the base 113 and the height (b) of the needle 114 to the pitch (c) of the needles 114 is preferably 0.3 or more and 1.1 or less.

### <Experimental Example 5>

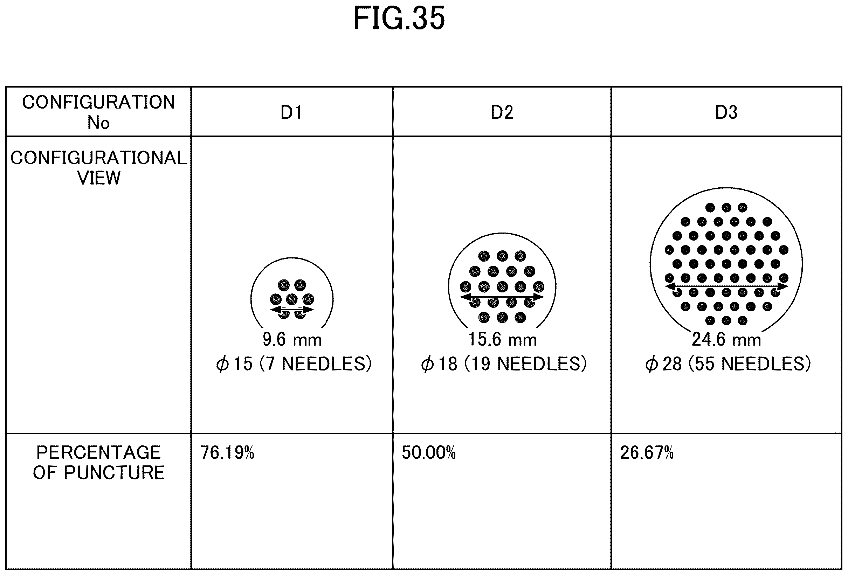

Puncture experiments were performed by providing a plurality of needles and not varying the pitch thereof. FIG. 35 is a table illustrating results of the puncture experiments when the plurality of base-equipped needles are arranged in rows, the number of needles is varied, and the pitch thereof is the same.

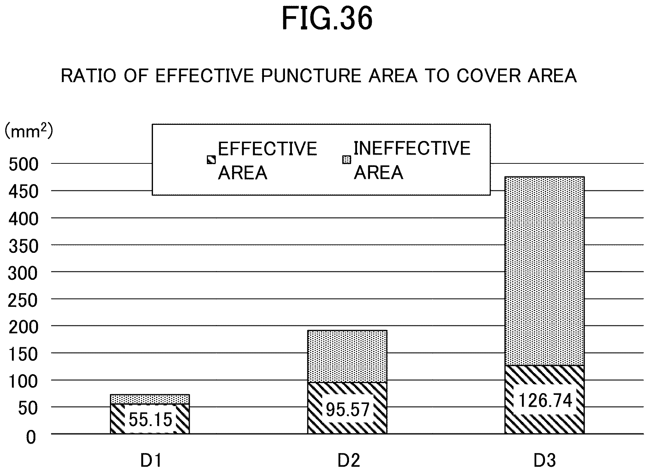

As illustrated in FIG. 35, when the pitch is the same, the percentage of puncture decreases as the number of needles increases. The effective puncture area was calculated by multiplying an assumed cover area with the percentage of puncture. The assumed cover area was calculated from the maximum diameter, indicated by an arrow, of a portion of the fine needle plate in which the base-equipped needles exist. FIG. 36 is a graph illustrating a ratio of the effective puncture area to the cover area of the fine needle plate of FIG. 35.

In order to increase the percentage of puncture of configurations D2 and D3 in which the plate substrate 111 of the fine needle plate is large and the percentage of puncture is low, it is conceivable to be effective to increase the load per needle at the time of the puncture. In this case, it is required to select a device configured to apply an appropriate quantity of force. Specifically, the forces applied to the target objects in this experiment were all 1,000 gf (1 kgf). In such a case, the forces applied to a single needle are calculated as follows: D1: 143 gf; D2: 53 gf; and D3: 18 gf.

When the force required for the puncture of 70% in D2 is calculated from the load applied to the single needle, the force can be calculated to be about 2,700 gf from the formula: 143 gf × 19 needles, in order to make D1 and D2 have the same percentage of the puncture. For example, the injection device 200 illustrated in FIG. 26, the needle applicator 300 illustrated in FIG. 27, or the injection device 6 illustrated in FIG. 28 can apply a force to a large area in response to pushing from the back side, and push the fine needle plate entirely, i.e., can apply the force more readily than in one finger. This is a conceivable reason why this force can be realized.

Also, the size of the discharge hole 14 (i.e., a through-hole), especially the size of the front end thereof, is set to an optimum value in accordance with the thickness of the needle 114. However, a tapered shape that gradually narrows from the back end to the front end is preferable. The diameter of the front end of the discharge hole 14 is preferably from about 5 um through about 100 pm, and more preferably from 10 um through 50 µm.

When the fine needle part of the first, third, or fourth embodiment is used in any of the application examples illustrated in FIG. 1 and FIGS. 25 to 30 described above, by integrally forming the needle and the base to have the above-described dimensions, the base pushes and stretches the surface of the target object, such as skin, and the skin is punctured by the needle. In this case, the percentage of puncture is increased even if a plurality of needles are arranged in an array. Thereby, liquid having some beneficial effect on the target object, e.g., a liquid composition or a liquid cosmetic containing any active ingredient, can be reliably delivered to the target object while pushing and stretching the target object, and can be retained in the interior of the target object near the surface thereof.

Also, even if the fine needle part of the second embodiment is used in any of the application examples illustrated in FIG. 1 and FIGS. 25 to 27 described above, by using a barrel including the needle-like projections formed on another plate so as to have the same dimensions as described above, the front end of the barrel pushes and stretches the surface of the target object, such as skin, and the skin is punctured by the needle-like projections. In this case, the percentage of puncture is increased even if a plurality of needles are arranged in an array.

The syringe, injecting device, and needle applicator, to which the fine needle plate or the fine needle part is attached, according to the above-described embodiments of the present invention have been described, with the content (liquid substance) being liquid. However, specifically, the content may be selected from the group consisting of a beauty substance, a cell suspension, a gel-like material, a therapeutic substance, and a diagnostic substance.

The beauty substance includes a typical beauty substance that can be contained in a cosmetic composition, and a beauty substance used for aesthetic medicine. Examples of the typical beauty substance can include: ascorbic acid or derivatives thereof, tranexamic acid, arbutin, 4-MSK (potassium 4-methoxysalicylate), and the like for skin lightening applications; and retinol, niacinamide, and hyaluronic acid or derivatives thereof, and the like for anti-wrinkle applications. However, these are by no means a limitation.

In addition to the typical beauty substance that can be contained in a cosmetic composition, examples of the beauty substance to be injected can include an adipocyte like a filler, hyaluronic acid, a botulinum toxin (Botox, Btx), and the like for wrinkle treatments. However, these are by no means a limitation.

Examples of the therapeutic substance include antibiotics, anesthetics, analgesics, vaccines, and antibodies. However, these are by no means a limitation.

Moreover, in order to inject, to a human subject, cells in a suspension or a liquid medium as the content housed in a syringe, the fine needle plate of the present invention may be used.

Moreover, the content to be housed in a syringe may be a mixture of a cell suspension and a growth factor. Alternatively, the cell suspension may contain a gel-like structure. Such a gel-like structure preferably represents a mixture of extracellular matrix proteins that mimic extracellular environments of different tissues, and further preferably a gel-like structure similar to hyaluronic acid.

Note that, in the above, the syringe, injecting device, or needle applicator in which the fine needle plate of the present invention is mounted has been described taking the human skin as an example of the target object to which the liquid is to be applied. The target object may be, for example, animal skin, or the surface of a plant culm, stem, or leaf. Alternatively, the target object may be tissue such as skin and organs that are taken out of a human subject or an animal subject.

While preferable embodiments of the present invention have been described above in detail, the present invention is not limited to the specific embodiments, and various modifications and changes are possible within the scope of the gist of the embodiments of the present invention described in the scope of claims.

The present international application claims priority to Japanese Patent Application No. 2022-056466, filed on March 30, 2022. The contents of Japanese Patent Application No. 2022-056466 are incorporated in the present international application by reference in their entirety.

### REFERENCE SIGNS LIST

1,1A,1B,1C,D,1α,2,2A,2B,2C,2D,3,3A,3B,4,4A,4B,4C Fine needle part
11,11B,11C,11D,11β,11Dδ Fine needle plate (solid type, needle plate)
111,111B,111C,111D Plate substrate
112 Two-step needle (solid)
113 Base
114 Needle (tip needle)
115 Lip portion
116 Discharge hole
118 Sealing valve
12,12B,12C,12D Attaching frame
123L Lip portion
131 Fine needle plate
21 Contact plate
316,216A,216B Barrel (base)
317 Space
22 Attaching frame
222 229,229A,229C Needle-like projection (needle)
31,31A Fine needle plate
311,311A Plate substrate
312 Two-step needle (hollow two-step needle)
313 Base
314 Needle (tip needle)
315 Discharge hole
316 Barrel
317 Space
32 Attaching frame
322 Flow path plate
41 Fine needle plate (solid type, needle plate)
411 Plate substrate
412,412A,412B,412C Lip-equipped two-step needle
413 Base
414 Needle (tip needle)
415 Individual lip
416 Discharge hole
42 Attaching frame
70 Applicator portion (puncture instrument)
80 Syringe (puncture instrument)
90 Injection mechanism (puncture instrument)
100 Syringe set
200 Injection device
300 Needle applicator
F13 Front recessed surface of base
F15 Front surface of individual lip

## Claims

1. A fine needle part to be attached to a skin puncture instrument, the fine needle part comprising:
a plate substrate having a plate shape;
a plurality of bases projecting from a surface of the plate substrate, the surface being situated so as to face a target object; and
a plurality of needles projecting from top surfaces of the plurality of bases, wherein
a height (b) of each of the plurality of needles is 5 µm ≤ b < 700 pm,
a height (a) of each of the plurality of bases is 200 µm ≤ a < 3,000 µm,
a total height (a+b) of the height (b) of each of the needles and the height (a) of each of the bases is from 600 um through 3,200 pm,
the plurality of bases and the plurality of needles are formed of a resin material that does not dissolve, and
by the target object being punctured by the needles, liquid is applied onto a surface of the target object or to an interior of the target object.

2. The fine needle part according to claim 1, wherein
the plate substrate, the plurality of bases, and the plurality of needles are integrally formed, and the plurality of bases and the plurality of needles form a plurality of two-step solid needles,
one or more discharge holes are formed in the plate substrate,
a lip portion is formed at an outer peripheral edge of a front surface of the plate substrate, and
by the liquid being pushed out from the discharge holes, the liquid spreads in a space formed by the target object, the lip portion, and the front surface of the plate substrate, and the liquid enters the interior of the target object through holes formed by the needles of the plurality of two-step solid needles.

3. The fine needle part according to claim 2, wherein
the plate substrate, the plurality of bases, and the plurality of needles are integrally formed, and the plurality of bases and the plurality of needles form the plurality of two-step solid needles,
one or more discharge holes through which fluid passes are formed in each of the top surfaces of the bases so as to vertically penetrate through the plate substrate and the bases,
a lip portion is provided at an outer peripheral edge of each of the top surfaces of the bases, and a recess is formed at a center of each of the top surfaces in a radial direction thereof, and
by the liquid being pushed out from the discharge holes, the liquid spreads in a space formed by the target object, the lip portion, and the recess of each of the top surfaces of the bases, and the liquid enters the interior of the target object through the holes formed by the needles of the plurality of two-step solid needles.

4. The fine needle part according to claim 1, wherein
the plate substrate, the bases, and the needles are integrally formed, and
a discharge hole that extends in a direction in which each of the needles projects and through which fluid passes is formed so as to vertically penetrate through the plate substrate, each of the bases, and each of the needles.

5. A fine needle part, comprising:
a flow path fine needle plate in which a flow path hole is formed; and
a contact plate disposed to face a front end of the flow path fine needle plate,
the flow path fine needle plate including
one or more needle-like projections projecting from a surface of the flow path fine needle plate at the front end, and
the contact plate including
an outer plate substrate disposed to face the front end of the flow path fine needle plate, and
one or more barrels projecting from an outer surface of the outer plate substrate, wherein
the barrels are disposed so as to be the same in number as the needle-like projections of the flow path fine needle plate and so as to be shorter than the needle-like projections such that the needle-like projections project from inner circumferences of the barrels of the contact plate, thereby forming two-step needles including bases that are the barrels and needles that are portions projecting from the barrels of the needle-like projections,
in the fine needle part, liquid passing through the flow path hole spreads in a space formed by the outer plate substrate of the flow path fine needle plate as accumulation of liquid, then the liquid enters gaps between the needle-like projections of the flow path fine needle plate and inner circumferential surfaces of the barrels and is discharged, and the liquid enters an interior of a target object through holes formed by the needle-like projections,
a height (b) of projection of each of the needle-like projections from the outer plate substrate is 5 µm ≤ b < 700 pm,
a height (a) of projection of each of the barrels from the outer plate substrate is 200 µm ≤ a < 3,000 um, and
a total height (a+b) of the height (b) of each of the needle-like projections and the height (a) of each of the barrels is from 600 um through 3,200 um.

6. The fine needle part according to claim 1, wherein
a value (b/a) of a ratio of the height (a) of each of the bases to the height (b) of each of the needles is 0.5 or less.

7. The fine needle part according to claim 1, wherein
a front-end diameter (d) of each of the bases is 200 µm < (d) ≤ 600 um.

8. The fine needle part according to claim 1, wherein
the plurality of bases are disposed apart from each other, and a pitch (c) of apexes of the needles is 1 mm ≤ c < 5 mm.

9. The fine needle part according to claim 1, wherein
a value ((a+b)/c) of a ratio of a total of the height (a) of each of the bases and the height (b) of each of the needles to a pitch (c) of the needles is 0.3 or more and 1.1 or less.

10. The fine needle part according to claim 8, wherein
an interval between peripheries of top surfaces of the bases is 0.5 mm or more and 4.4 mm or less.

11. The fine needle part according to claim 1, wherein
a number of the bases per unit area is 0.1/cm² or more and 33/cm² or less.

12. The fine needle part according to claim 1, further comprising:
a frame,
the frame including
a lip portion formed at an outer peripheral edge of a surface on a front side thereof,
the plate substrate, the plurality of bases, and the plurality of needles are integrally formed, and the plurality of bases and the plurality of needles form a plurality of two-step solid needles, and
by the liquid being pushed out from a gap between an inner periphery of the lip portion of the frame and an outer periphery of the plate substrate, the liquid spreads in a space formed by the target object, the lip portion, and a front surface of the plate substrate, and the liquid enters the interior of the target object through the holes formed by the plurality of two-step solid needles.

13. The fine needle part according to claim 12, wherein
the plate substrate has a cylindrical shape and includes the bases and the needles on an outer side thereof.

14. An injection device, comprising:
the fine needle part of any one of claims 1 to 13; and
a skin puncture instrument to which the fine needle part is attached and that is configured to house the liquid.

15. A puncture injection set, comprising:
the injection device of claim 14;
a beauty tool including a grasping tool to be grasped by a user; and
a housing container in which the liquid is housed, wherein
the target object is punctured by the needles after or before application of the liquid housed in the housing container to the target object, thereby applying the liquid onto the surface of the target object or to the interior of the target object.
